# EUROPEAN PATENT SPECIFICATION

(11) **EP 1 336 104 B1**
(45) Date of publication and mention of the grant of the patent: **25.03.2009**
(21) Application number: 01992891.0
(22) Date of filing: 01.11.2001
(51) Int. Cl.: G01N 33/543, C12Q 1/68, G01N 33/68, G01N 30/02

(54) **PROTEOMICS BASED DRUG COMPARISON METHODS**
PROTEOMANALYSE VERFAHREN ZUM DROGEN VERGLEICH
PROCEDES D'ANALYSE PROTEOMIQUE DE COMPARAISON DES DROGUES

(30) Priority: 01.11.2000 US 245035 P
(43) Date of publication of application: 20.08.2003
(73) Proprietor: Minerva Biotechnologies Corporation, Newton, MA 02458 (US)
(72) Inventor: BAMDAD, Cynthia, C., Newton, MA 02458 (US); BAMDAD, R., Shoshana, Newton, MA 02458 (US)
(74) Representative: Killin, Stephen James
(86) International application number: PCT/US2001/044964
(87) International publication number: WO 2002/037109

(56) References cited:
- WO-A-00/43783
- WO-A-00/43791
- WO-A-00/54882
- WO-A-00/63694
- WO-A-00/63701
- WO-A-02/01225
- WO-A-02/01230
- WO-A-02/28507
- WO-A-02/29411
- WO-A-99/38013
- US-A- 4 791 069

## Description

### FIELD OF THE INVENTION

This invention relates generally to methods for the rapid and sensitive detection of the interaction of chemical and/or biological species. Techniques including drug screening are facilitated by the invention.

### BACKGROUND OF THE INVENTION

The recent elucidation of the human genome has produced a vast amount of information in the form of discrete DNA sequences, which encode the repertoire of human proteins. As is appreciated by those familiar with molecular biology, it is the proteins, not the DNA or RNA, which are the predominant molecules involved in biological function. In functional genomics, altered levels of particular mRNAs are correlated to a disease state. Proteomics is the successor to functional genomics because it studies the function of the proteins rather than their precursor molecules, DNA or mRNA. One aspect of proteomics is determining how protein function is correlated to disease. Disease-related families of proteins, or proteins involved in a common signaling pathway, can be identified by elucidating protein-protein interaction networks. A major focus of biological study, today, is the elucidation of the protein interaction networks that make up vital signal transduction pathways. Critical clues, as to what triggers transformation from the healthy state to the disease state, are being gleaned from understanding these protein interaction networks.

A major impediment to the study of proteomics is the lack of available methods to detect protein-protein interactions when one or both are as yet uncharacterized or impure. Most existing detection methods require the use of specific antibodies that recognize one or both of the putative binding partners. This means that the proteins of interest need to be purified so that antibodies can be raised against them. If a protein has been characterized, it can be genetically labeled with an affinity tag, which often aids the detection process. This means that prior to knowing whether or not a crude sample contains a protein of interest, much work must be done to separate and purify components such that they may be used in existing assays. Another major drawback of existing methods for the study of protein-protein interactions is that they are sequential, labor-intensive processes. This means that they cannot be multiplexed to address complex problems such as elucidating large protein interaction networks or testing several putative binding partners, in parallel. With the number of genes in the human genome now estimated to be about 40K, determining interaction networks by sequential pair-wise testing will involve approximately 8X10⁸ experiments. With the near completion of the human genome project, it is imperative that adequate technologies be developed to enable the functional analysis of the many gene products. For these reasons, it would be advantageous if methods were available that facilitated the parallel analysis of protein binding interactions, in which one or both proteins are may be present in a crude mixture. It would also be advantageous if methods were available to rapidly characterize newly discovered, uncharacterized proteins, which could be generated for example from a cDNA library. These methods would be of particular importance in various industries, including for example, the pharmaceutical industry, where large quantities of known and unknown species are screened to identify new drugs. In addition to identification, it may also be useful to categorize species as to their relative affinity to other known or unknown species. Typically, to increase the total number species screened, it is often desirable to increase the speed with which the screening procedure takes place. In addition, sample throughput is improved when detection methods can be simplified and when multiple assays can be run concurrently.

WO0063701A discloses proteomics methods for differential expression profiles upon exposure to a drug.

### SUMMARY OF THE INVENTION

In an aspect of the invention, there is provided a method of comparing the activities of a first drug or drug candidate and a second drug or drug candidate, comprising:
providing a first surface having a plurality of individually spatially addressable regions, wherein a different protein or protein interaction motif is fastened to each of the regions;
treating a first sample comprising cellular components with the first drug or drug candidate;
exposing the treated first sample to the first surface, and allowing interaction to occur between the components and each of the regions, such that at least one of the components binds to at least one of the protein or protein interaction motifs, thereby defining a first interaction pattern between the components and each of the regions;
determining the first interaction pattern;
providing a second surface having a plurality of individually spatially addressable regions that are identical to those of the first surface;
treating a second sample comprising cellular components, which are identical to those of the first sample, with the second drug or drug candidate;
exposing the treated second sample to the second surface, and allowing interaction to occur between the components and each of the regions, such that at least one of the components binds to at least one of the protein or protein interaction motifs, thereby defining a second interaction pattern between the components and each of the regions;
determining the second interaction pattern; and
comparing the first and second interaction patterns.

Other advantages, novel features, and objects of the invention will become apparent from the following detailed description of the invention when considered in conjunction with the accompanying drawings, which are schematic and which are not intended to be drawn to scale. In the figures, each identical or nearly identical component that is illustrated in various figures is represented by a single numeral. For purposes of clarity, not every component is labeled in every figure, nor is every component of each embodiment of the invention shown where illustration is not necessary to allow those of ordinary skill in the art to understand the invention.

### BRIEF DESCRIPTION OF THE DRAWIIVGS

Figure 1 depicts a surface that is divided into separate parts with a different binding species attached to each part of the surface. Colloid particles in suspension are selectively interacting with the species attached to one part of the surface and not with the other.
Figure 2 shows the surfaces depicted in Figure 1 after unbound colloid particles have been rinsed from the surface.

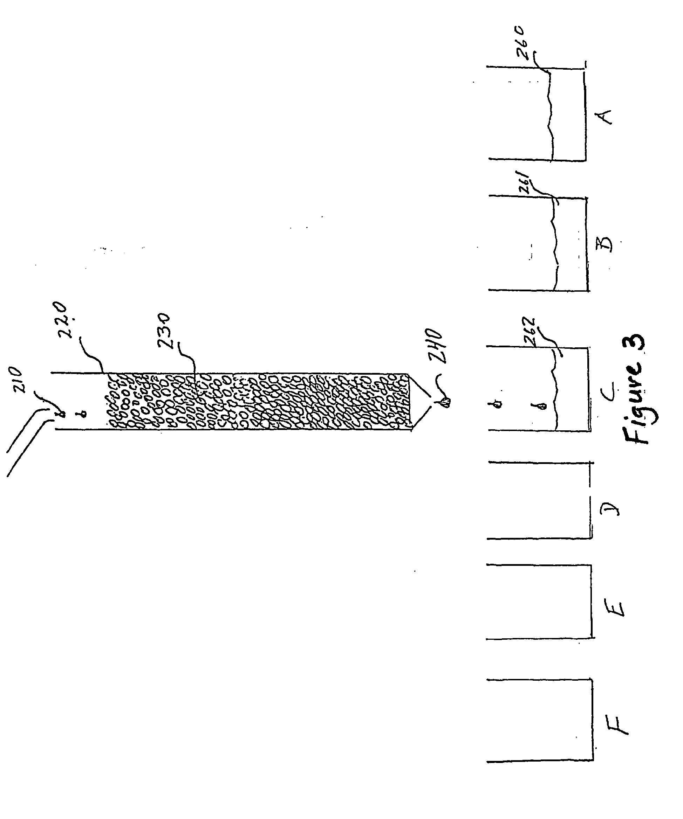

Figure 3 illustrates schematically a mixture being separated in a separation column with successive aliquots of the different components from the mixture being collected sequentially.
Figure 4 illustrates schematically the addition of colloid particles, including suspect binding partners, into the aliquots obtained from the procedure shown in Figure 3.
Figure 5 is a photocopy of a photograph (40-fold magnification) of colloid-decorated beads, specifically, colloids linked to beads via protein/protein interaction.

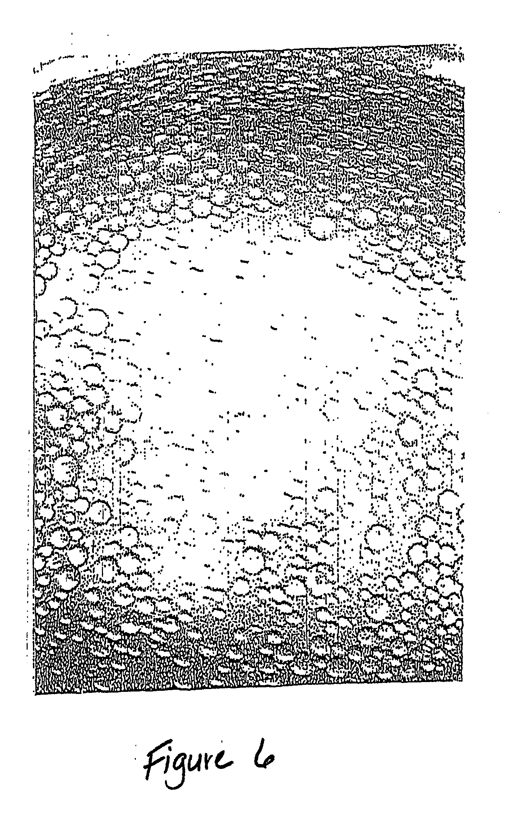

Figure 6 is a photocopy of a photograph (40-fold magnification) of the negative control of the experiment shown in Figure 7.
Figure 7 illustrates a multi-well plate providing separately addressable assay regions.
Figure 8 is a photocopy of a photograph of gold colloids displaying a small molecule agglomerating onto beads displaying a cognate protein and coloring the beads red.
Figure 9 is a photocopy of a photograph of the negative control for the experiment shown in Figure 8 in which the bead displayed a random protein and not the binding partner of the small molecule presented on the colloid surface.
Figure 10 is a photocopy of a photograph of an experiment in which a single bead displaying the cognate protein was mixed into a background of beads displaying a random protein and shows the selective agglomeration of colloids onto that single bead.
Figure 11 is a photocopy of a fluorescent micrograph of an experiment in which colloids displayed both a binding partner of a species immobilized on a bead and a fluorescent moiety.
Figure 12 is a photocopy of photographs of multiple sets of a protein-protein binding experiment in which a first protein was colloid-immobilized and the second was bead-immobilized.
Figure 13 is a photocopy of photographs of multiple sets of a protein-protein binding experiment in which a first protein was colloid-immobilized and the second was bead-immobilized directly from a whole cell lysate.
Figure 14 is a photocopy of a photograph of SAM-coated colloids displaying a fluorescently labeled antibody, anti-GST, binding to its cognate ligand, GST, which was immobilized on an NTA-SAM-coated chip.
Figure 15 is photocopy of a photograph of the comparison experiment to that shown in Figure 14 in which fluorescently labeled anti-GST, free in solution, bound to its cognate ligand, GST, which was immobilized on an NTA-SAM-coated chip.

### DETAILED DESCRIPTION OF THE INVENTION Definitions

"Small molecule", as used herein, means a molecule less than 5 kiloDalton, more typically less than 1 kiloDalton. As used herein, "small molecule" excludes proteins.

The term "candidate drug" as used herein, refers to any medicinal substance used in humans, animals, or plants. Encompassed within this definition are compound analogs, naturally occurring, synthetic and recombinant pharmaceuticals, hormones, antimicrobials, neurotransmitters, etc. This includes any substance or precursor (whether naturally occurring, synthetic or recombinant) which is to be evaluated for use as a drug for treatment of neurodegenerative disease, or other disease characterized by aberrant aggregation, or prevention thereof. Evaluation typically takes place through activity in an assay, such as the screening assays of the present invention.

A variety of types of particles can be used in the invention. For example, "fluid suspendable particle" means a particle that can be made to stay in suspension in a fluid in which it is used for purposes of the invention (typically an aqueous solution) by itself, or can be maintained in solution by application of a magnetic field, an electromagnetic field, agitation such as stirring, shaking, vibrating, sonicating, centrifuging, vortexing, or the like. A "magnetically suspendable" particle is one that can be maintained in suspension in a fluid via application of a magnetic field. An electromagnetically-suspendable particle is one that can be maintained in suspension in a fluid by application of an electromagnetic field (e.g., a particle carrying a charge, or a particle modified to carry a charge). A "self-suspendable particle" is a particle that is of low enough size and/or mass that it will remain in suspension in a fluid in which it is used (typically an aqueous solution), without assistance of for example a magnetic field, for at least 1 hour. Other self-suspendable particles will remain in suspension, without assistance, for 5 hours, 1 day, 1 week, or even I month, in accordance with the invention.

"Proteins" and "peptides" are well-known terms in the art, and are not precisely defined in the art in terms of the number of amino acids that each includes. As used herein, these terms are given their ordinary meaning in the art. Generally, peptides are amino acid sequences of less than about 100 amino acids in length, but can include sequences of up to 300 amino acids. Proteins generally are considered to be molecules of at least 100 amino acids.

As used herein, a "metal binding tag" refers to a group of molecules that can become fastened to a metal that is coordinated by a chelate. Suitable groups of such molecules include amino acid sequences including, but not limited to, histidines and cysteines ("polyamino acid tags"). Metal binding tags include histidine tags, defined below.

As used herein, "chelate coordinating a metal" or metal coordinated by a chelate, refers to a metal coordinated by a chelating agent that does not fill all available coordination sites on the metal, leaving some coordination sites available for binding via a metal binding tag.

As used herein, "metal binding tag/metal/chelate linkage" defines a linkage between first and second species in which a first species is immobilized relative to a metal binding tag and a second species is immobilized relative to a chelate, where the chelate coordinates a metal to which the metal binding tag is also coordinated. U.S. Patent No. 5,620,850 of Bamdad, et al., describes exemplary linkages.

"Signaling entity" means an entity that is capable of indicating its existence in a particular sample or at a particular location. Signaling entities used in the invention can be those that are identifiable by the unaided human eye, those that may be invisible in isolation but may be detectable by the unaided human eye if in sufficient quantity (e.g., colloid particles), entities that absorb or emit electromagnetic radiation at a level or within a wavelength range such that they can be readily determined visibly (unaided or with a microscope including an electron microscope or the like), or spectroscopically, entities that can be determined electronically or electrochemically, such as redox-active molecules exhibiting a characteristic oxidation/reduction pattern upon exposure to appropriate activation energy ("electronic signaling entities"), or the like. Examples include dyes, pigments, electroactive molecules such as redox-active molecules, fluorescent moieties (including, by definition, phosphorescent moieties), up-regulating phosphors, chemiluminescent entities, electrochemiluminescent entities, or enzyme-linked signaling moieties including horse radish peroxidase and alkaline phosphatase. "Precursors of signaling entities" are entities that by themselves may not have signaling capability but, upon chemical, electrochemical, electrical, magnetic, or physical interaction with another species, become signaling entities. An example includes a chromophore having the ability to emit radiation within a particular, detectable wavelength only upon chemical interaction with another molecule. Precursors of signaling entities are distinguishable from, but are included within the definition of, "signaling entities" as used herein.

As used herein, "fastened to or adapted to be fastened", in the context of a species relative to another species or to a surface of an article, means that the species is chemically or biochemically linked via covalent attachment, attachment via specific biological binding (e.g., biotin/streptavidin), coordinative bonding such as chelate/metal binding, or the like. For example, "fastened" in this context includes multiple chemical linkages, multiple chemical/biological linkages, etc., including, but not limited to, a binding species such as a peptide synthesized on a polystyrene bead, a binding species specifically biologically coupled to an antibody which is bound to a protein such as protein A, which is covalently attached to a bead, a binding species that forms a part (via genetic engineering) of a molecule such as GST or Phage, which in turn is specifically biologically bound to a binding partner covalently fastened to a surface (e.g., glutathione in the case of GST), etc. As another example, a moiety covalently linked to a thiol is adapted to be fastened to a gold surface since thiols bind gold covalently. Similarly, a species carrying a metal binding tag is adapted to be fastened to a surface that carries a molecule covalently attached to the surface (such as thiol/gold binding) which molecule also presents a chelate coordinating a metal. A species also is adapted to be fastened to a surface if a surface carries a particular nucleotide sequence, and the species includes a complementary nucleotide sequence.

"Covalently fastened" means fastened via nothing other than one or more covalent bonds. E.g. a species that is covalently coupled, via EDC/NHS chemistry, to a carboxylate-presenting alkyl thiol which is in turn fastened to a gold surface, is covalently fastened to that surface.

"Specifically fastened (or bound)" or "adapted to be specifically fastened (or bound)" means a species is chemically or biochemically linked to another specimen or to a surface as described above with respect to the definition of "fastened to or adapted to be fastened", but excluding all non-specific binding.

"Non-specific binding", as used herein, is given its ordinary meaning in the field of biochemistry.

"Colloids", as used herein, means nanoparticles, i.e. very small, self-suspendable or fluid-suspendable particles including those made of material that is, e.g., inorganic or organic, polymeric, ceramic, semiconductor, metallic (e.g. gold), non-metallic, crystalline, amorphous, semiconductor nanocrystals, or a combination. Typically, colloid particles used in accordance with the invention are of less than 250 nm cross section in any dimension, more typically less than 100 nm cross section in any dimension, and in most cases are of about 2-30 nm cross section. One class of colloids suitable for use in the invention is 10-30 nm in cross section, and another about 2-10 nm in cross section. As used herein this term includes the definition commonly used in the field of biochemistry.

As used herein, a component that is "immobilized relative to" another component either is fastened to the other component or is indirectly fastened to the other component, e.g., by being fastened to a third component to which the other component also is fastened, or otherwise is translationally associated with the other component. For example, a signaling entity is immobilized with respect to a binding species if the signaling entity is fastened to the binding species, is fastened to a colloid particle to which the binding species is fastened, is fastened to a dendrimer or polymer to which the binding species is fastened, etc. A colloid particle is immobilized relative to another colloid particle if a species fastened to the surface of the first colloid particle attaches to an entity, and a species on the surface of the second colloid particle attaches to the same entity, where the entity can be a single entity, a complex entity of multiple species, a cell, another particle, etc.

The term "sample" refers to any medium suspected of containing an analyte, such as a binding partner, the presence or quantity of which is desirably determined. A sample can be a biological sample such as a cell, cell lysate, tissue, serum, blood or other fluid from a biological source, a biochemical sample such as products from a cDNA library, an environmental sample such as a soil extract, or any other medium, biological or non-biological, including synthetic material, that can advantageously be evaluated in accordance with the invention.

A "structurally predetermined sample", as used herein means samples, the chemical or biological sequence or structure of which is a predetermined structure used in an assay designed to test whether the structure is associated with a particular process such as a neurodegenerative disease. For example, a "structurally predetermined sample" includes a peptide sequence, random peptide sequence in a phage display library, and the like.

A "sample suspected of containing" a particular component means a sample with respect to which the content of the component is unknown. The sample may be unknown to contain the particular component, or may be known to contain the particular component but in an unknown quantity.

As used herein, a "metal binding tag" refers to a group of molecules that can become fastened to a metal that is coordinated by a chelate. Suitable groups of such molecules include amino acid sequences, typically from about 2 to about 10 amino acid residues. These include, but are not limited to, histidines and cysteines ("polyamino acid tags"). Such binding tags, when they include histidine, can be referred to as a "poly-histidine tract" or "histidine tag" or "HIS-tag", and can be present at either the amino- or carboxy-terminus, or at any exposed region, of a peptide or protein or nucleic acid. A poly-histidine tract of six to ten residues is preferred for use in the invention. The poly-histidine tract is also defined functionally as being a number of consecutive histidine residues added to a protein of interest which allows the affinity purification of the resulting protein on a metal chelate column, or the identification of a protein terminus through the interaction with another molecule (e.g. an antibody reactive with the HIS-tag).

A "moiety that can coordinate a metal", as used herein, means any molecule that can occupy at least two coordination sites on a metal atom, such as a metal binding tag or a chelate.

"Affinity tag" is given its ordinary meaning in the art. Affinity tags include, for example, metal binding tags, GST (in GST/glutathione binding clip), and streptavidin (in biotin/streptavidin binding). At various locations herein specific affinity tags are described in connection with binding interactions. It is to be understood that the invention involves, in any embodiment employing an affinity tag, a series of individual embodiments each involving selection of any of the affinity tags described herein.

"Molecular wires" as used herein, means wires that enhance the ability for a fluid encountering a SAM-coated electrode to communicate electrically with the electrode. This includes conductive molecules or, as mentioned above and exemplified more fully below, molecules that can cause defects in the SAM allowing communication with the electrode. A non-limiting list of additional molecular wires includes 2-mercaptopyridine, 2-mercaptobenzothiazole, dithiothreitol, 1, 2-benzenedithiol, 1, 2-benzenedimethanethiol, benzene-ethanethiol, and 2-mercaptoethylether. Conductivity of a monolayer can also be enhanced by the addition of molecules that promote conductivity in the plane of the electrode. Conducting SAMs can be composed of, but are not limited to: 1) poly (ethynylphenyl) chains terminated with a sulfur; 2) an alkyl thiol terminated with a benzene ring; 3) an alkyl thiol terminated with a DNA base; 4) any sulfur terminated species that packs poorly into a monolayer; 5) all of the above plus or minus alkyl thiol spacer molecules terminated with either ethylene glycol units or methyl groups to inhibit non specific adsorption. Thiols are described because of their affinity for gold in ready formation of a SAM. Other molecules can be substituted for thiols as known in the art from U.S. Patent No. 5,620,820, and other references. Molecular wires typically, because of their bulk or other conformation, create defects in an otherwise relatively tightly-packed SAM to prevent the SAM from tightly sealing the surface against fluids to which it is exposed. The molecular wire causes disruption of the tightly-packed self-assembled structure, thereby defining defects that allow fluid to which the surface is exposed to communicate electrically with the surface. In this context, the fluid communicates electrically with the surface by contacting the surface or coming in close enough proximity to the surface that electronic communication via tunneling or the like can occur.

The term "biological binding" refers to the interaction between a corresponding pair of molecules that exhibit mutual affinity or binding capacity, typically specific or non-specific binding or interaction, including biochemical, physiological, and/or pharmaceutical interactions. Biological binding defines a type of interaction that occurs between pairs of molecules including proteins, nucleic acids, glycoproteins, carbohydrates, hormones and the like. Specific examples include antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc.

The term "binding" or "bound" refers to the interaction between a corresponding pair of molecules that exhibit mutual affinity or binding capacity, typically specific or non-specific binding or interaction, including biochemical, physiological, and/or pharmaceutical interactions. Biological binding defines a type of interaction that occurs between pairs of molecules including proteins, nucleic acids, glycoproteins, carbohydrates, hormones and the like. Specific examples include antibody/antigen, antibody/hapten, enzyme/substrate, enzyme/inhibitor, enzyme/cofactor, binding protein/substrate, carrier protein/substrate, lectin/carbohydrate, receptor/hormone, receptor/effector, complementary strands of nucleic acid, protein/nucleic acid repressor/inducer, ligand/cell surface receptor, virus/ligand, etc.

The term "binding partner" refers to a molecule that can undergo binding with a particular molecule. Biological binding partners are examples. For example, Protein A is a binding partner of the biological molecule IgG, and vice versa.

The term "determining" refers to quantitative or qualitative analysis of a species via, for example, spectroscopy, ellipsometry, piezoelectric measurement, immunoassay, electrochemical measurement, and the like. "Determining" also means detecting or quantifying interaction between species, e.g. detection of binding between two species.

The term "self-assembled monolayer" (SAM) refers to a relatively ordered assembly of molecules spontaneously chemisorbed on a surface, in which the molecules are oriented approximately parallel to each other and roughly perpendicular to the surface. Each of the molecules includes a functional group that adheres to the surface, and a portion that interacts with neighboring molecules in the monolayer to form the relatively ordered array. See Laibinis, P. E.; Hickman, J.; Wrighton, M. S.; Whitesides, G. M. Science 245, 845 (1989), Bain, C.; Evall, J.; Whitesides, G. M. J. Am. Chem. Soc. 111, 7155-7164 (1989), Bain, C.; Whitesides, G. M. J. Am. Chem. Soc. 111, 7164-7175 (1989).

The term "self-assembled mixed monolayer" refers to a heterogeneous self assembled monolayer, that is, one made up of a relatively ordered assembly of at least two different molecules.

Certain embodiments of the invention make use of self-assembled monolayers (SAMs) on surfaces, such as surfaces of colloid particles, and articles such as colloid particles having surfaces coated with SAMs. In one set of preferred embodiments, SAMs formed completely of synthetic molecules completely cover a surface or a region of a surface, e.g. completely cover the surface of a colloid particle. "Synthetic molecule", in this context, means a molecule that is not naturally occurring, rather, one synthesized under the direction of human or human-created or human-directed control. "Completely cover" in this context, means that there is no portion of the surface or region that directly contacts a protein, antibody, or other species that prevents complete, direct coverage with the SAM. I.e. in preferred embodiments the surface or region includes, across its entirety, a SAM consisting completely of non-naturally-occurring molecules (i.e. synthetic molecules). The SAM can be made up completely of SAM-forming species that form close-packed SAMs at surfaces, or these species in combination with molecular wires or other species able to promote electronic communication through the SAM (including defect-promoting species able to participate in a SAM), or other species able to participate in a SAM, and any combination of these. Preferably, all of the species that participate in the SAM include a functionality that binds, optionally covalently, to the surface, such as a thiol which will bind to a gold surface covalently. A self-assembled monolayer on a surface, in accordance with the invention, can be comprised of a mixture of species (e.g. thiol species when gold is the surface) that can present (expose) essentially any chemical or biological functionality. For example, they can include tri-ethylene glycol-terminated species (e.g. tri-ethylene glycol-terminated thiols) to resist non-specific adsorption, and other species (e.g. thiols) terminating in a binding partner of an affinity tag, e.g. terminating in a chelate that can coordinate a metal such as nitrilotriacetic acid which, when in complex with nickel atoms, captures a metal binding tagged-species such as a histidine-tagged binding species. In this way, the present invention provides a method for rigorously controlling the concentration of essentially any chemical or biological species presented on a colloid surface or any other surface. In many embodiments of the invention the self-assembled monolayer is formed on gold colloid particles.

A "kit" means a series of components that are designed and constructed to be used together, e.g. in a single assay. Components of a kit are arranged in combination, e.g. packaged together.

A "package" means a container that maintains its content separate from other containers or its surroundings. For example, a kit defined by a first package including a first component and a second package including a second component may include two separate packages containing the respective components, or may include a single unit that includes two sections within which the respective components can be maintained in isolation from each other (e.g. prior to their use).

"Stationary phase," as used herein, is used in a manner consistent with the meaning applied by those skilled in the art of chromatographic science. For example, a stationary phase may be any stationary phase used in column, liquid, gas or supercritical fluid chromatography. Examples include alumina, silica gel and ion exchange resin. "Stationary phase" includes homogeneous solid substances or an underlying support coated with, for example, a liquid phase.

### DETAILED DESCRIPTION OF THE INVENTION

Related applications include International patent application serial number PCT/US00/01997, filed 01/25/00 by Bamdad et al., entitled "Rapid and Sensitive Detection of Aberrant Protein Aggregation in Neurodegenerative Diseases" (published as WO 00/43791 on 07/27/00), International patent application serial number PCT/U500/01504, filed 01121/00 by Bamdad, et al, entitled "Interaction of Colloid-Immobilized Species with Species on Non-Colloidal Structures" (published as WO 00/43783 on 07/27/00), commonly-owned, copending U.S. patent application serial no. 09/602,778, filed 06/23/00 by Bamdad et al., entitled "Interaction of Colloid-Immobilized Species with Species on Non-Colloidal Structures"; and commonly-owned, copending U.S. patent application serial no. 09/631,818, filed 08/03/00 by Bamdad et al., entitled "Rapid and Sensitive Detection of Protein Aggregation".

Interactions can be detected between two binding partners wherein one binding partner is attached to a nanoparticle and the other is attached to a surface. Surfaces used may be any type of solid support capable of fastening to a binding species or adaptable to fastening to the species, including, but not limited to, microtitre plates, chips, glass plates, electrodes, beads and other colloids. The surface may be a sensing surface that generates a signal when binding occurs, or, alternatively, the surface may present the attached species to available colloid particles and the binding interaction may be observed without the use of an auxiliary signaling capability. For example, when the surface is another colloid, the colloid need not carry an auxiliary signaling element. The binding of the two immobilized species on the two colloids may draw the colloids together, which results in a change in the solution color from pink to blue. However, in some cases it may be desirable for the colloid to carry an auxiliary signaling entity that may be detected.

Methods are described herein in which the interaction of two binding partners is detected using a variety of methods, which may include detecting an inherent optical property of the nanoparticles to which the binding partners have been attached, or by detecting a signaling entity that has been attached to one or both of the binding partners.

Both the putative binding partner and the auxiliary signaling entity may be attached to a common substrate, such as to a common nanoparticle. Alternatively, it is the agglomeration of the colloidal gold particles onto a surface that is detected. Colloidal gold particles that bear a binding species will agglomerate onto a surface that presents its cognate binding partner, and the agglomeration of the gold particles may color that surface red. Alternatively, putative binding species may be attached to particles made out of semiconductor material, such as semiconductor nanocrystals of silicon or gallium arsenide. In this case each putative binding species is attached to a set of nanoparticles of a different size. These nanoparticles differentially fluoresce depending on the size of the particle. In this way, one may be able to determine which particle-immobilized species bound to the surface-immobilized species by detecting a particular fluorescent signal indicative of the particle size, which is then correlated to the attached binding species.

Putative binding species and a signaling entity may be attached to a common nanoparticle. Examples of signaling entities that can be attached to nanoparticles include but are not limited to fluorescent moieties, fluorescent proteins, enzymes, dyes and redox-active moieties that can deliver an electronic or electrochemical signal. Binding interactions between species on particle and species on surfaces are then detected by detecting the particle-attached signaling entity.

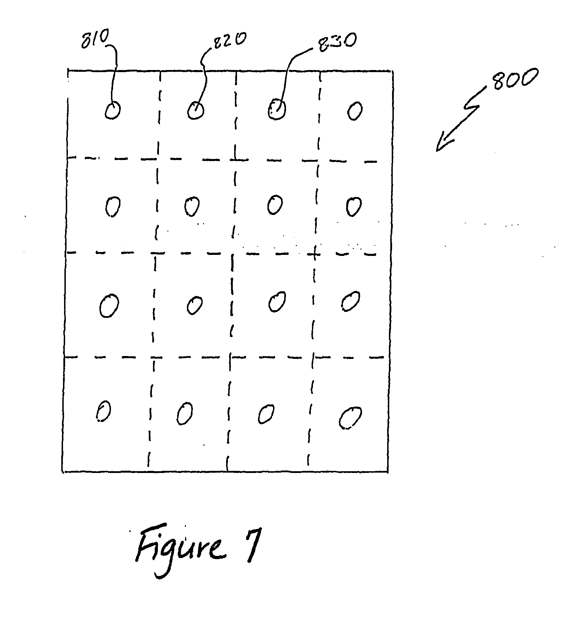

A binding species may be fastened to a single surface or to a variety of surfaces or to different portions on a single surface. To facilitate high screening throughput, the surface may include individually spatially addressable regions, such as different wells of a multi-well plate, a plurality of electrodes, regions on a surface, or discrete regions on a chip. For example, a plurality of electrodes might be arranged in individual wells of a multi-well plate or a chip may be divided into a matrix so that multiple samples may be tested concurrently on a single surface such as a chip. Figure 7 is an illustration of a multi-well plate including discrete wells, 810, into which different samples may be placed for discrete analysis of each sample.

Binding partners need not be attached or immobilized to surfaces, per se. For example, binding partners can be fastened to, for example, a polymer, dendrimer, or hapten, which may or may not also bear signaling entities.

As is appreciated by those skilled in the art, it may not always be possible or practical to purify putative binding species or to derivatize them with affinity tags, to facilitate surface attachment, prior to performing biological binding assays. It would therefore be a significant improvement if methods were available to detect binding events between a target species and putative binding partners that are present in a crude mixture. As described herein, it is possible to separate the components of a mixture and attach the separated components to solid substrates or to different regions of a solid substrate, such as a spatially addressable chip. Putative binding partners of one or more types that have been attached to colloid particles may then be introduced to the separated components, and interactions between the separated components and the putative binding partners may be determined.

The components of a mixture can also be separated and the separated components attached to colloid particles. Putative binding partners of one or more types that exist on or have been attached to a surface may then be introduced to the colloid particles that present the separated components, and interactions between the separated components and the putative binding partners may be determined.

The components of a mixture can also be separated through the use of chromatography. The separated components that are contained in the eluent from the chromatographic column are collected in distinct locations and fastened to surfaces. These surface-attached components are then introduced to colloids, which have been derivatized to present putative binding partners. Binding interactions between colloid-immobilized species and surface-bound species are then determined by detecting either an inherent property of the colloid or by detecting the presence of a signaling element that has also been attached to the colloidal particle. Alternatively, the eluted components can be immobilized on colloids and interaction between the colloids and a surface presented with either known or unknown binding partners can be determined.

A mixture may be separated into two or more components. The mixture may be any substance that may contain a binding partner or other compound of interest. The components that are separated from the mixture may be binding partners of a known species or an uncharacterized species, such as products of cDNA libraries, a protein, a receptor, a nucleic acid, an antibody, or a drug, and may be attached onto a surface and tested for interactions with colloid particles that may include drugs or other chemical or biological species.

A crude mixture may be separated by chromatography. Eluted fractions may be separately collected in vials that contain beads that are designed to capture the separated components either through non-specific adsorption or through a specific interaction. The vials may be designed such that there are two compartments that are separated by a dialysis membrane. The upper compartment contains beads and receives the eluted fraction. The lower compartment is exposed to a reservoir to facilitate buffer exchange. As the elution buffer is dialyzed against a binding buffer, the buffer is altered and the separated components of the mixture become attached to the beads.

The beads may be of the same or a different type as is used in the chromatography column. Several techniques for attaching biological and chemical species to a bead, through adsorption or covalent attachment, are known to those of skill in the art. A selection of beads derivatized with chemical functionalities to facilitate coupling protocols and beads presenting binding partners of commonly used affinity tags, such as glutathione, NTA-Ni, biotin and the like are commercially available.

The beads, which now present putative binding species and are still retained in separate vials, are then exposed to nanoparticles that carry immobilized species, which may be binding partners of the bead-immobilized species.

The binding of the nanoparticle-immobilized species to the bead-immobilized species is detected by detecting an inherent property of the nanoparticles or by detecting an attached signaling entity. For example, if the nanoparticles that are used are gold colloids, the binding of the colloids to the beads is clearly visible as the agglomeration of the gold particles onto the bead colors the bead red. Alternatively, the nanoparticle can be derivatized to present an auxiliary, detectable signaling element. Signaling entities may include fluorescent moieties, naturally fluorescent proteins, electroactive or redox active moieties, such as ferrocene derivatives, or signaling enzymes such as horseradish peroxidase or up-regulating phosphors.

The signaling entity and a putative binding species may be both attached to a common nanoparticle.

The signaling entity may be incorporated into a self-assembled monolayer that is formed on the surface of the nanoparticle. Alternatively, binding interactions are detected by detecting an intrinsic property of the nanoparticle. Nanoparticles that are formed from semiconductor material, differentially fluoresce depending on the size of the particle.

The species can also be adsorbed non-specifically onto the surface of chromatography beads, for example, ion exchange resin. Adsorption beads may include those incorporating Dextran, DEAE, q-sepharose, agarose and polystyrene. Adsorption beads may be coated with a charged species to facilitate fastening to a wide variety of binding partners. Beads providing for specific attachment may present, for example, NTA, streptavidin, biotin, glutothione, protein A, and protein G. Beads can provide a surface to which binding partners may be conveniently fastened as well as provide a substrate on which colloid particle decoration may be quickly and easily determined. Beads may also provide a vehicle for conveying fastened colloids to a second surface, such as an electrode, by gravity without a need for additional recruiting forces.

Reagents may be added to the vials that contain the beads and the chromatographically separated components, to facilitate the binding of the component to the bead. For example, agents can be added to the fraction to alter the pH of the solution. Chemical coupling agents such as EDC/NHS can be added to facilitate covalent linkage of the component to beads presenting, for example, carboxylates.

Components that have been chromatographically separated can be attached to different locations on a spatially addressable surface or chip and interactions detected as described herein. The surface can be substantially flat so that a homogeneous population of colloids bearing a putative binding partner can be incubated with the surface and binding of colloids to a particular location is detected. Alternatively, the surface can be contoured to facilitate incubation of each location with a separate colloid mixture. Each location of the surface may present the same or a different component that may bind to species attached to the colloid particles.

As another option, a second colloid particle may be used. Fractions can be collected in separate locations and introduced to colloids that have been adapted to facilitate binding of the separated component to the colloid surface. For example, colloids derivatized with self-assembled monolayers that display exposed carboxylates can be covalently linked to components having an exposed amine using coupling chemistry such as EDC/NHS. Colloids bearing antibodies may be used to bind a targeted species to its surface. Colloids modified with self-assembled monolayers bearing nitrilo tri-acetic acid groups and nickel can be used to selectively bind histidine-tagged species, including, but not limited to, His-tagged peptides and nucleic acids.

The source of the species being assayed may be a purified component for which the binding partner is unknown and must be identified from a large pool of candidates. For example, a drug may be known to have a therapeutic effect for a particular disease state, but the target of its action may be unknown. In this case, the drug is attached to a colloid particle that may or may not bear auxiliary signaling capabilities. To identify its binding partner(s), cells or tissues derived from a source associated with that particular disease state can be processed to yield a mixture of cell-derived biomolecules. Components of the mixture may then be separated by a variety of known techniques including column chromatography. Elution fractions, that contain either purified components or a mixture containing far fewer components, are collected separately. The elution buffer can be altered by, for example, exchanging or diluting the fluid to form a suitable binding buffer, and components from separated fractions may be separately adhered to solid supports that are preferably, but not necessarily, spatially addressable. A solution containing colloid particles bearing the drug may then be incubated with the solid supports bearing the putative binding partners. Any binding of the colloid to a particular solid support can be detected and the immobilized binding partner analyzed, using techniques known to those of ordinary skill in the art, to determine its identity.

Alternatively, the source of the species being assayed may be a mixture, such as a cell lysate, a soil sample, a plant extract, any mixture containing natural products, herbal mixtures, or a water sample. For example, it may be known that a particular biomolecule is essential for an identified disease process, and it is therefore advantageous to find an agent that will bind to and block the action of this disease-associated biomolecule. In this case, the disease-associated biomolecule can be attached to a set of colloid particles that may or may not bear auxiliary signaling entities. A crude mixture, which may contain medicinal substances, can then be separated as described above, adhered to beads or other solid substrates and separately tested for their ability to interact with the colloid-bound species. Without the use of methods described herein, this can be a lengthy, expensive and labor-intensive process.

Similarly, if a mixture is suspected to have medicinal properties, the target of action within cells or tissues may be determined in a similar manner. The non-specific fastening methods of the invention may allow presentation of uncharacterized binding partners. In this manner, vast quantities of unknown proteins and other biologically active compounds from natural samples can be quickly screened for specific biological and chemical activity.

Once a mixture is obtained, it may be separated into different components, preferably through chromatography. The chromatographic procedures used may be those known in the art. A variety of different substrates or stationary phases may be used to separate the components of the mixture, including, for example, size exclusion beads and adsorption beads. For instance, the components of interest in a cell lysate may be adsorbed onto a stationary phase in a chromatography column and selectively eluted off of the column based on differences such as size, charge to mass ratio, and structure. This may be done by altering the mobile phase through the varying of factors such as pH, salt content, and ionic strength. Successive aliquots of eluent may be collected, with each aliquot containing a species or a group of species that may be unique. The species contained in each aliquot may then be fastened to a surface. If an aliquot contains more than one binding species of interest, it may be further separated under different conditions or with a different column to achieve further separation of the different species.

After aliquots of the different components of the mixture have been obtained, suspected binding partners that may be contained in those aliquots can be re-bound to a solid substrate such as the surfaces described above.

The species may be adsorbed onto beads of the same type used to perform the chromatographic separation. As the samples taken off of the column will be in a non-binding elution buffer, it is preferred that the buffer be altered by, for example, exchanging to a binding buffer so that the species of interest can be adsorbed onto the beads. This may be accomplished through a dialysis membrane which allows for the exchange of elution buffer for binding buffer. Once the buffer has been exchanged, beads may be added to each aliquot and the species of interest may be adsorbed or reacted onto the surface. Beads may also be added prior to exchanging the buffer. Preferably, few beads are used so that the surface density of the species on the beads is increased.

The beads, with species of interest immobilized to them, may be incubated with colloid particles to determine if there is an interaction between the colloid particles and the surfaces.

Ready-to-use sets of particles can be generated to which putative binding partners have been or can be separately attached. These sets of particles constitute a probe library that may be incorporated into a kit for use in diagnostics or drug screening. This library of species-presenting colloids can then be used to probe, for example: 1) tissue specimens or cells for the presence of molecules or structures associated with a particular disease state; 2) biochips presenting a single or a multitude of putative binding partners; 3) solutions containing known or unknown biomolecules attached to colloids or beads; or 4) any or all of the above in the presence or absence of a drug candidate.

A kit can have a first package that contains colloid particles which are fastened to a first species as well as a second package that contains colloid particles which are fastened to a second species. The kit may be used to detect binding partners for the first species, the second species, or both, either sequentially or in parallel. Alternatively, the kit may contain discrete sets of colloids, pre-bound with specific agents, in multi-well plate format. In this case the kit would also provide a set of colloids upon which the user can immobilize a particular protein of interest, which the user provides. The user may then add an aliquot of the protein of interest to each well of the multi-well plate and determine binding partners of the protein of interest. Species which can be colloid immobilize include, for example, any nucleic acid, protein, peptide, antibody, components generated by cDNA libraries, the afore-mentioned protein interaction motifs, drug candidates, enzymes and the like.

Components of the probe library may be generated using methods described herein. For example, a mixture can be chromatographically separated, and derivatized colloids can be added to the vials that hold the fractions. Alternatively, the fractions can be added to sets of colloids that have been derivatized to facilitate the attachment of the fractionated components. The colloids, which may now present putative binding partners are then packaged and made available as a part of a kit.

A kit can include particles, which have been derivatized to facilitate the attachment of putative binding partners, and instructions for immobilizing a putative binding partner.

Methods are described herein that may facilitate the systematic characterization of proteins of unknown function by determining the ability of the protein of interest to bind to known species. Such known species may include, but are not limited to, chemical compounds, drugs or drug candidates, cells, proteins, protein domains, peptides and nucleic acids, including unnatural variations thereof such as PNAs or peptides made up of D-amino acids.

Techniques for the detection of binding partners and drug candidates using colloid particles are described in commonly-owned, copending U. S. patent application serial no. 09/631,818, filed 08/03/00 by Bamdad et al., entitled "Rapid and Sensitive Detection of Protein Aggregation" and in International Patent Application Serial No. PCT/US00/01997 by Bamdad et al. To facilitate efficient drug screening and to narrow classes of suspect compounds using these techniques, it may be useful to perform assays that can screen for a variety of species simultaneously or in batches. In addition, once a particular substance is identified, additional useful information may be obtained if the particular component of the substance can be isolated and identified as a specific target.

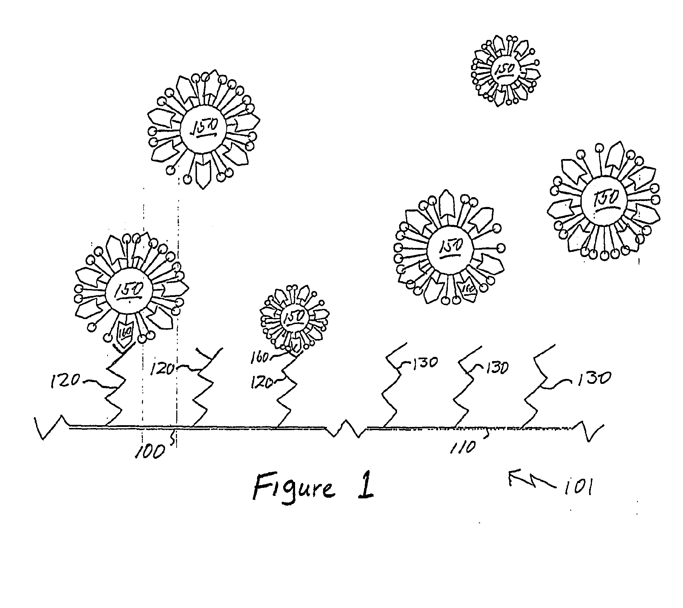

Figure 1 provides an illustration of a surface 101 that is divided into discrete regions, 100 and 110. Attached to region 100 is a first binding species 120. Attached to region 110 is a second binding species 130 that is different from binding species 120. Colloid particles 150 including binding partner 160 are incubated over the entire surface 101. After adequate incubation, binding has occurred between binding species 160 and 120, thus fixing a portion of the colloid particles 150 to surface 100. No binding has occurred between binding species 160 and 130 and thus no colloid particles are fixed to surface 110. The bound colloid particles are observable on the surface 100.

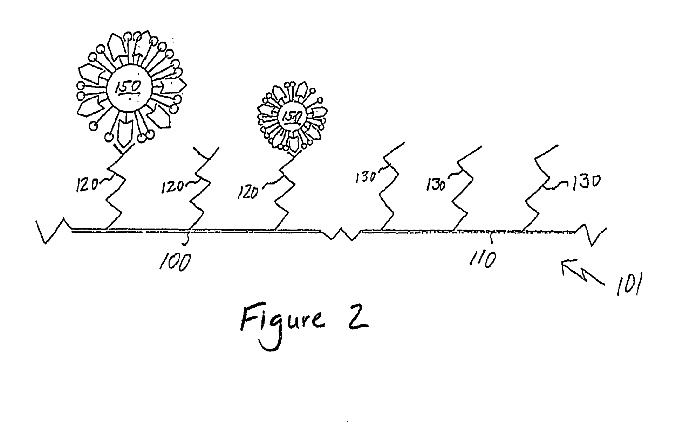

Figure 2 shows the same surfaces as Figure 1 after unbound colloid particles have been rinsed off. Colloid particles remain attached to surface 100 but no decoration has occurred on surface 110. This provides an observable difference between the two surfaces. Rinsing may not be necessary, depending on the detection method that is used.

Certain embodiments of the invention make use of self-assembled monolayers (SAMs) formed on the surfaces of colloid particles. Preferably, all of the species that participate in the SAM include a functionality that binds, optionally covalently, to the surface, a functionality that resists non-specific adsorption, and a functionality that facilitates the attachment of biomolecules or drug candidates. In a preferred embodiment a self-assembled monolayer is formed on a gold colloid. A self-assembled monolayer, whether formed on a colloidal particle or on another surface, can be comprised of a mixture of thiol species (when gold is the surface) that includes tri-ethylene glycol-terminated thiols to resist non-specific adsorption and thiols terminating in a binding partner of an affinity tag, e.g. terminating in a chelate that can coordinate a metal such as nitrilo tri-acetic acid which, when in complex with nickel atoms, capture histidine-tagged binding species. Similarly, self-assembled monolayers can be formed on particles comprised of silicon or semiconductor material.

Examples of solid substrates that may be used include beads as well as planar substrates such as chips. A chip may be any uniformly coated substrate to which a binding partner may be fastened. The chip may be coated with gold or silicon, which can be readily modified to facilitate the fastening of a species. The chip may include a self-assembled monolayer (SAM) which may either be conductive or non-conductive and the chip may also be an electrode or a series of discrete electrodes. If a SAM is used on a chip, it may incorporate binding partners of convenient affinity tags, including nucleic acids, or functional groups to facilitate covalent coupling of species to the surface, for example via EDC/NHS chemistry.

In an embodiment of the invention, an electrode, which can define a portion of a PC board, may be used as a surface, and a SAM may be formed on gold-coated pads of the PC board. One method of forming a SAM on a PC board is to coat regions of the board with an adhesive layer of, for example, titanium or nickel, and then to apply a gold coating. The SAM may then be applied to the gold layer as described above.

An "interaction finger print" of a sample, mixture or compound may be created. These interaction finger prints may then be compared to other fingerprints to determine similarities or differences between the two samples. Two or more separate surfaces or separate regions on a surface, can be derivitized and presented with one or more components such as a biomolecular array. Single compounds, drugs, drug candidates, cell lysates, mixtures of drug candidates, etc., may be exposed to the two or more surfaces and the interaction characteristic between a component in the sample and with one or more regions on the surface can be determined. Interaction characteristics may range from no binding to complete binding and may also include values anywhere in between. As the different addressable regions on the surface or surfaces may present different putative binding partners to the sample, results may be different at each of the addressable locations. By recording the amount of binding interaction at multiple addressable locations, a "contour map" or "interaction pattern" of binding interaction can be produced. The interaction pattern may be referred to as three dimensional in that the "z" axis component may be represented by the magnitude of the interaction characteristic at or close to an addressable location. This interaction pattern, showing different degrees of interaction at each of the addressable regions, can be recorded and later compared to similar interacting patterns produced from the same, similar or different surfaces when exposed to the same or a different sample. Thus, a similar three dimensional interaction landscape may be indicative of similar composition, or for example, similar drug activity. Once specific interaction patterns are generated, they may form a library to which any newly generated interaction patterns may be compared.

Described herein are methods for the controlled and specific attachment of chemical or biological species to colloids. For example, a SAM can be used to fasten binding partners to the colloidal particles, to the solid substrates, or both. The use of a SAM provides an efficient, specific method of fastening a variety of binding partners to colloids. One method of covalent attachment using a SAM is via EDC/NHS chemistry. Another method uses a chelate/metal/metal binding tag linkage. In an arrangement where a metal binding tag/metal/chelate linkage is used, a chelate can form part of a SAM on a colloid, and can be covalently attached to a signaling entity. The chelate coordinates a metal, but leaves at least two open coordinate sites on the metal. A metal binding tag such as a polyamino acid tag can be incorporated into the binding species, giving the binding species the ability to fasten to the colloid or signaling entity by coordination of the metal binding tag to the metal. Examples of suitable chelates include nitrilotriacetic acid, 2,2'-bis(salicylideneamino)-6,6'demethyldiphenyl, or 1,8-bis(a-pyridyl)-3,6-dithiaoctane. In an alternate immobilization technique, binding species can carry a terminal cysteine and fasten thereby to a gold surface of the colloid.

Virtually any biological species can be immobilized on either colloidal particles or solid supports. For example, a SAM may be formed on the surface of the colloid and a variety of species may be attached to the SAM. Some of the methods that can be used to form a SAM are described in U.S. Patent No. 5,620,850, and in U.S. patent application serial no. 09/602,778, filed 06/23/00 by Bamdad et al., entitled "Interaction of Colloid-Immobilized Species with Species on Non-Colloidal Structures"; and in commonly-owned, copending U.S. patent application serial no. 09/631,818, filed 08/03/00 by Bamdad et al., entitled "Rapid and Sensitive. Detection of Protein Aggregation". A SAM may include a thiol terminated with Nitrilotriacetic Acid Ni⁺⁺ (NTA/Ni⁺⁺) residue (or triethyleneglycol terminated thiols) that can be used to effectively capture histidine-tagged proteins. SAMs may also incorporate carboxylic acid groups allowing for the coupling of unmodified proteins through the use of standard EDC/NHS chemistry. The proteins need not be labeled as they may be attached to a labeled component. In addition, a SAM may include moieties used for signaling that a binding event has occurred. Signaling moieties may be, for example, charged, electro-active, or fluorescent compounds that serve to increase the detectability of an interaction involving a binding partner. Metallocenes, such as ferrocene, are an example of a signaling entity that may help to increase the sensitivity of the invention. Colloid particles may include a number of different signaling entities as well as a number of different binding species.

As discussed above, a first binding partner may be immobilized to a surface such as a chip or bead and a second binding partner may be immobilized to a colloid particle. Both the first binding partner and the second binding partner may be either known or unknown. For example, a known species may be immobilized on the surface of a bead while a series of unknown species, for example, separated components from a mixture, are immobilized on a series of colloids to be followed by an assay in which binding interaction between the unknown species on the colloid particles and the known species on the bead surface is determined. Alternatively, the binding partner immobilized on the colloid particles may be known while the binding partner that has been immobilized on the bead surface is unknown. Various permutations of this procedure are also available, such as where the binding partners on both the colloid particles and the bead surface are unknown or where the binding partners on both the colloid particles and the bead surface are known.

Competitive assays are possible; for example, a drug or putative drug that is not immobilized on a colloid or on a surface may be incubated along with a colloid particle that contains a binding species that may compete for binding sites on the surface. In this manner, the binding activity of the unattached drug may be judged by an absence of binding between the colloid particle and the exposed surface. Thus, a positive result may be indicated by an absent or depressed signal.

A variety of colloids fastened to different binding partners may be assayed with other binding partners affixed to one or more surfaces. If the signaling method of each of the colloids being tested is different, then a binding event between any one of the colloid types on the surface may be selectively determined. For example, one colloid particle may include a moiety that emits light at a first wavelength, and a second colloid type may include a moiety that emits light at a second wavelength. After an appropriate incubation, the surface may be examined for colloid attachment. If light of the first wavelength is present at the surface above a background threshold, then the first colloid type has been successfully bound to the surface, but if the light detected is of the second wavelength, then successful binding has occurred between the second colloid type and the surface. This type of analysis may be used competitively to determine which colloid fastened binding species is most readily bound to the binding species affixed to the surface or, alternatively, it may be used as a screening technique to concurrently test the binding activity of two or more binding species with a surface presenting a third or many binding species. By monitoring a change in signal over time, the dynamics of a binding interaction may be studied and recorded. The colloid particles used in this assay may or may not include a signaling moiety, and the binding efficiencies of the different colloid particles may be observable through a difference in color, shape, size or other detectable feature of the decorated surface.

### EXAMPLES

### Example 1

This example describes methods for forming SAMs on colloids as well as planar substrates. Surfaces can be coated with self-assembled monolayers for selective attachment of essentially any species as follows.

In one specific technique, initially, 1.5 ml of commercially available gold colloid (Auro Dye) is pelleted by centrifugation in a microfuge on high for 10 minutes. The pellet is resuspended in 100 µL of the storage buffer (sodium citrate and Tween-20). 100 µL of a dimethyl formamide (DMF) solution containing 90 µM nitrilo tri-acetic acid (NTA)-thiol, 90 µM ferrocene-thiol, and 500 µM carboxy-terminated thiol is then added. Following a 3-hour incubation in the thiol solution, the colloids are pelleted and the supernatant discarded. The colloids are then incubated in 100 µL of 400 µM tri-ethylene glycol-terminated thiol in DMF for 2 minutes at 55°C, 2 minutes at 37 °C, 1 minute at 55 °C, 2 minutes at 37 °C, then room temperature for 10 minutes. The colloids are then pelleted and 100 µl of phosphate buffered saline (PBS) is added. The colloids are then diluted 1:1 with 180 µM NiS04 in the colloid storage buffer. 100 µL of a His-tagged peptide at 100 µM in PBS is added to 100 µL of NTA-Ni(II) presenting colloids and is incubated for 0.5 hours. To remove free, unattached peptide, the colloids are then pelleted and the supernatant discarded. The colloid pellet is then resuspended in 100 µL PBS.

One method of forming a SAM on a planar substrate is as follows: Glass microscope slides are sputtered with a layer of Ti followed by a layer of Au. Each electrode is incubated at room temperature for times ranging from 60 seconds to several hours with 300. µL of a DMF solution that contained 10% methyl-terminated thiol (HS-(CH₂)₁₅ CH₃), 40% tri-ethylene glycol-terminated thiol, HS(CH₂)₁₁(CH₂CH₂)₃OH, (formula) and 50% poly (ethynylphenyl) thiol (C₁₆H₁₀S). 2 ml of 400 µM tri-ethylene glycol-terminated thiol is then added to a scintillation vial containing the chip and the vial is heat cycled in a water bath as follows: 2 minutes @ 55°C; 2 minutes @ 37°C; 1 minute @ 55°C; 2 minutes @ 37°C then RT for 10 min. Electrodes are then dipped in EtOH, then sterile PBS to rinse.

### Comparative Example 1

In this experiment gold colloids displaying a small molecule specifically bound to beads that presented a protein that the small molecule recognized. The agglomeration of the gold colloids onto the beads colored them red such that the binding event was readily detected by direct observation.

Beads that were coated with streptavidin were purchased commercially. Colloids were derivatized with self assembled monolayers as in Example 1 above. The SAMs that were formed on these colloids incorporated thiols terminated with i) tri-ethylene glycol to resist non-specific binding, ii) biotin, which specifically binds to streptavidin, and iii) nitrilo tri-acetic acid complexed with Ni⁺⁺ (NTA-Ni⁺⁺), which captures histidine-tagged proteins.

Colloids that presented NTA-Ni alone and did not present biotin were prepared to be used as a negative control.

Into each well of a microtitre plate was added 5 µL of the commercially available streptavidin coated beads. Into one well was added a single streptavidin bead and 5 µL of beads coated with Protein A. 25 µL of 0.01 mg/mL of a histidine-tagged glutathione-S-transferase (GST) protein was added to 100 µL of NTA-Ni/biotin presenting colloids. 25 µL, of his-tagged GST was added to colloids that presented NTA-Ni alone (no biotin) for use as a negative control. 80 µL of the colloid solution was added to wells containing the beads.

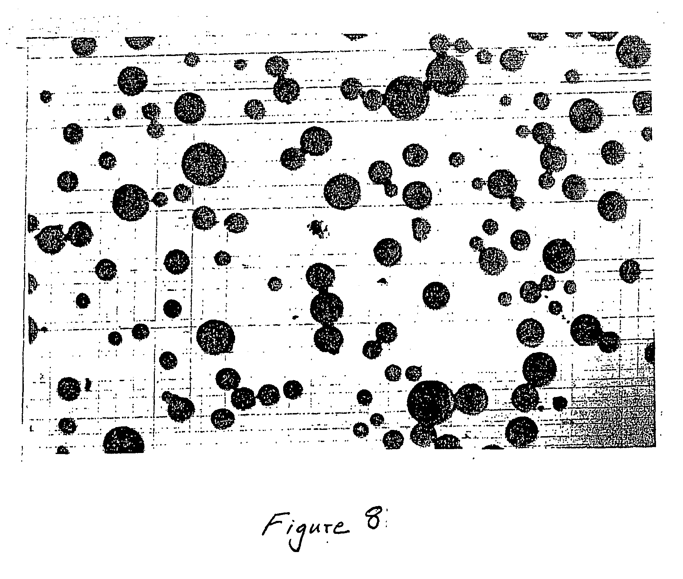

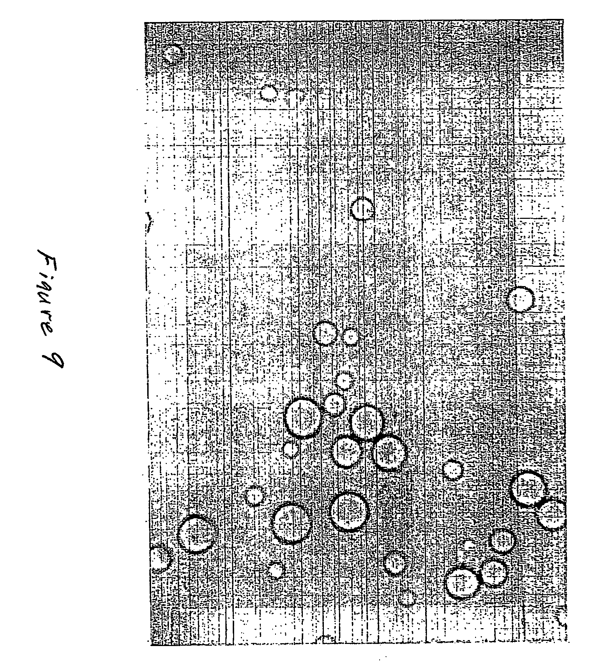

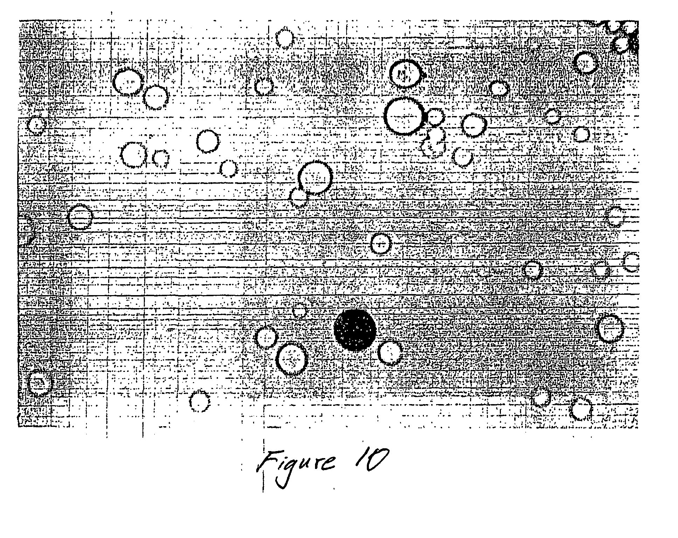

After 2 hours, colloids that displayed biotin had agglomerated onto the streptavidin beads and colored them bright red and the background pink color of the colloid solution had disappeared; see Figure 8. The solution in the wells that contained streptavidin beads and colloids that displayed NTA-Ni, but not biotin, appeared pink. Under 40-fold magnification it could be seen that the beads remained colorless, indicating that binding between species on the colloids and species on the beads had not taken place, see Figure 9. Figure 10 shows that in the well that contained the single streptavidin bead, in a background of Protein A beads, was bound by the biotin-presenting colloids while neighboring control beads were not.

### Comparative Example 2

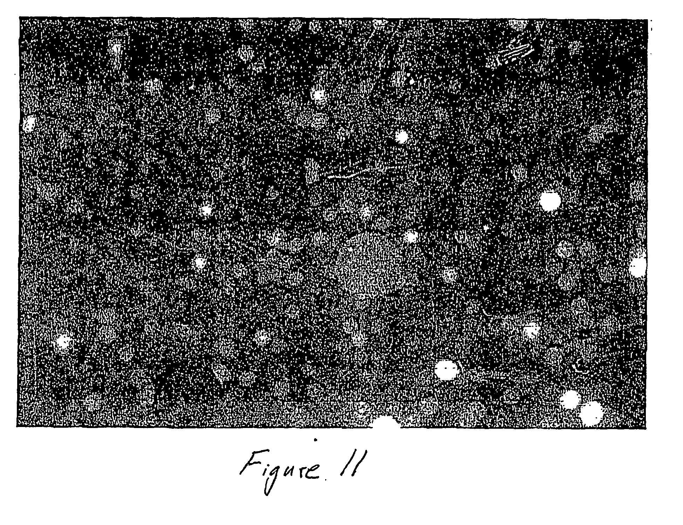

The beads used in the experiment described above were then used to demonstrate that bead-colloid interactions can also be detected using fluorescent moieties attached to the colloids. The colloids described above were derivatized to bear histidine-tagged GST. An anti-GST antibody that carried a fluorescent label was separately added to the colloids, rinsed with PBS, then added to the streptavidin or control beads. Beads were then visualized under a fluorescent microscope at 40-fold magnification. Figure 11 shows that the beads, as well as free-floating colloids, fluoresce. The control beads did not fluoresce at all.

### Comparative Example 3

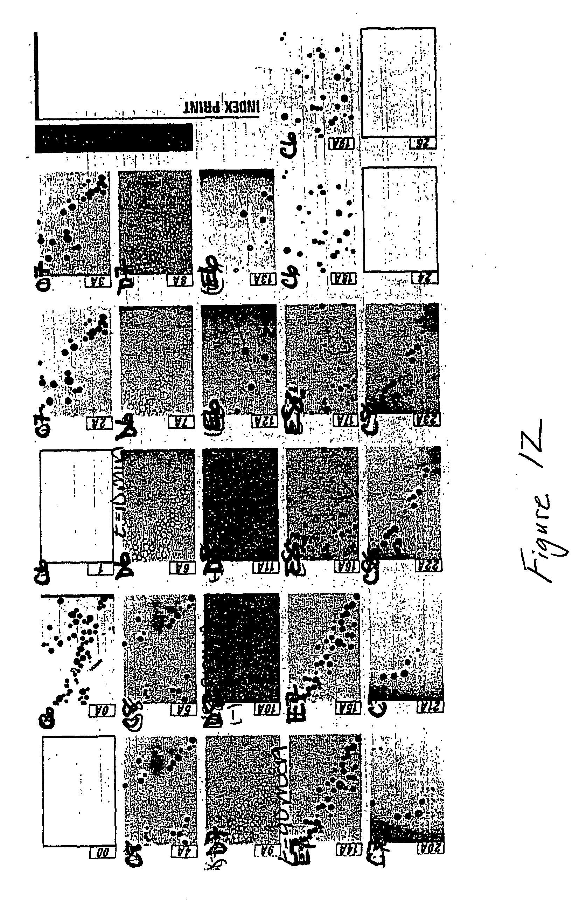

This experiment demonstrates the detection of an interaction between two proteins when the first is immobilized on beads and the second is immobilized on SAM-coated gold colloids. A histidine-tagged GST was attached to commercially available NTA-Ni⁺⁺ beads as follows. 100 µL of NTA-Ni⁺⁺ beads were rinsed in PBS then resuspended in 200 µL of PBS. 20 µL of the slurry was removed to a clean eppendorf tube to which was added 100 µL of His-tagged GST at 1 mg/ml. An aliquot of NTA-Ni⁺⁺ beads that had not been incubated with GST was reserved for use as a negative control. After a 40-minute incubation period, the beads were rinsed in PBS to remove excess protein, pelleted by centrifugation and then resuspended in 100 µL. Colloids were derivatized with SAMs that presented NTA-Ni⁺⁺ to capture a histidine-tagged protein and tri-ethylene glycol to aid in resisting non-specific binding: 500 µL of the colloids were then incubated with 100 µL of a solution containing 0.01 mg/mL of a histidine-tagged fragment of Protein G, which captures antibodies by binding to their Fc portion. Colloids were rinsed and pelleted to remove excess Protein G. The Protein G-presenting colloids were then incubated with 100 µL of an anti-GST antibody at 1 mg/mL. The colloids were rinsed with PBS. The anti-GST-presenting colloids were then mixed with the GST-presenting beads and binding was allowed to occur. Within 10 minutes, beads that presented GST began to become colored red as the antibody-bearing colloids agglomerated onto the beads. Referring now to Figure 12, the photos marked C6, C7 and C8 correspond to positives; beads presenting GST incubated with colloids presenting anti-GST. Photos labeled D6, D7, and D8 correspond to the negative controls, which were colloids presenting anti-GST incubated with NTA beads that were not pre-bound with GST. Photos labeled E6, E7 and E8 are again positives and correspond to beads that present GST, mixed with colloids that present anti-GST. The difference between series C and E is that the colloids used in series E were incubated with 10 µL of Protein G rather than 100 µL as was used in the beads pictured in series C.

### Comparative Example 4

This experiment demonstrates the detection of an interaction between a first species immobilized on SAM-coated colloids and a second species immobilized on beads, wherein the second species is directly bead-immobilized from a whole cell lysate.

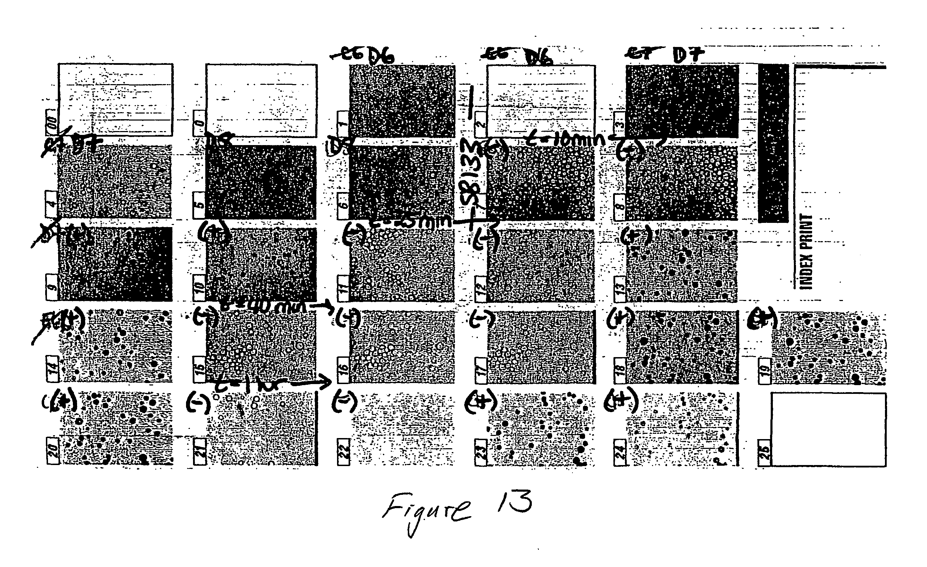

Competent BL21 cells were transformed with an expression plasmid coding for His-tagged GST. The cells were grown, used to inoculate LB and induced to express the encoded protein, as is familiar to those skilled in standard molecular biology techniques. For use as a negative control, an aliquot of BL21 cells were not transformed with an expression plasmid and were grown and induced as above with the exception that they were not grown in the presence of the selection antibody. Cells were pelleted and lysed by mixing with a standard lysis buffer followed by sonication. Commercially available NTA-Ni⁺⁺ beads were added to the crude cell lysate and incubated for 1 hr at 4 degrees to allow the His-tagged protein to bind to the beads. Beads were then pelleted, resuspended in PBS and mixed with colloids that presented anti-GST (prepared as described above). After an incubation period of 1 hour, beads were examined under 40-fold magnification. Results were photographed and are shown in Figure 13. Results of this experiment begin where the sheet is labeled "SB 133 10 min.". Each photo is labeled with a "+" or "-" and with the incubation time. Positives correspond to cells that were transformed with a GST expression plasmid and negatives correspond to cells that were not transformed, but otherwise treated the same.

### Comparative Example 5

This example demonstrates the detection of an interaction between a first protein species attached to a planar chip and a second species attached to a colloid, which also presents a fluorescent moiety. Herein, the binding of chip-immobilized GST to a fluorescently labeled antibody, either attached to colloids or free in solution, is compared.

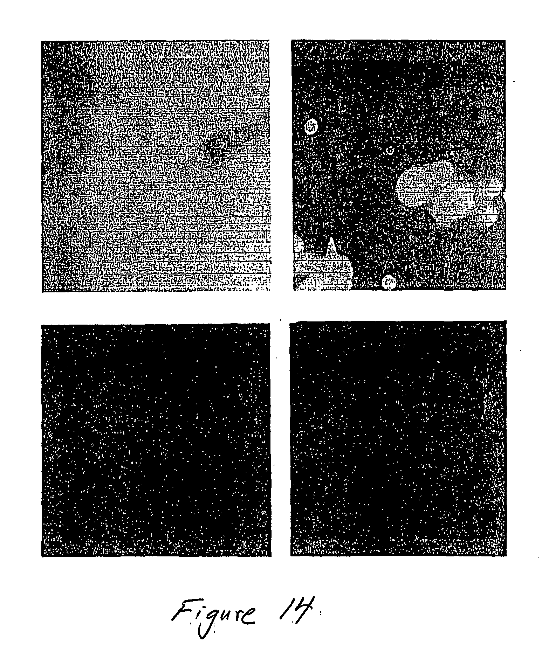

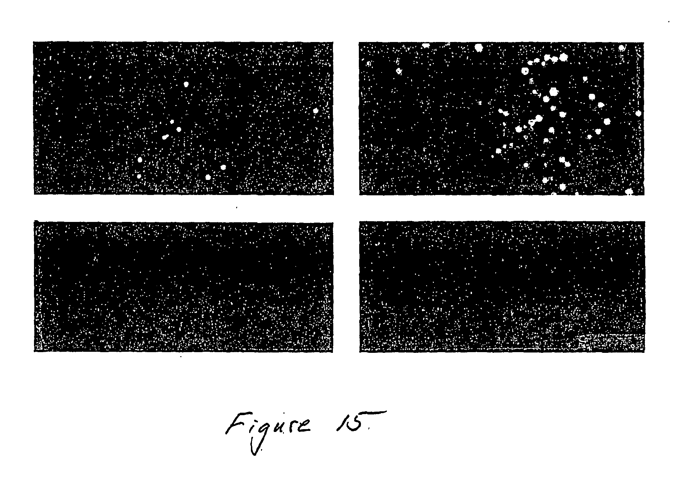

SAMs were formed on gold-coated planar chips such that 2.5% NTA-Ni⁺⁺ was presented in a background of 97.5% tri-ethylene glycol. A series of NTA-Ni⁺⁺ chips was pre-bound with His-tagged GST. NTA-Ni⁺⁺-SAM coated colloids were prepared and pre-bound with His-tagged Protein G, then incubated with a green fluorescein labeled anti-GST antibody. Half of the GST-presenting chips were incubated with the fluorescent antibody-bearing colloids, (see top of Figure 14) while the other half was incubated directly with the fluorescent anti-GST (see top of Figure 15). The controls (bottom half of Figures 14 and 15) are chips that were derivatized with tri-ethylene glycol terminated SAMs and did not present NTA- Ni⁺⁺. For each figure there are two positives, which correspond to two different concentrations of anti-GST added. Chips were analyzed and photographed under 40-fold magnification on a fluorescent microscope.

### Comparative Example 6 (Prophetic)

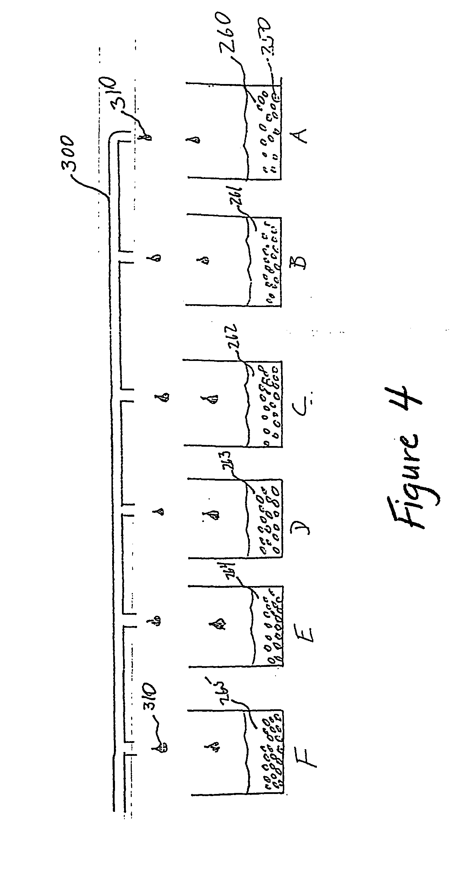

An extract from a soil sample purported to contain a drug candidate is adsorbed onto a chromatography column 220 as illustrated in Figure 4. Chromatography column 220 contains polystyrene resin beads 230 which adsorb species of interest that may be dissolved or suspended in extract 110. After the compounds have been suspended on the beads, an elution solvent is passed through the column and the pH of the elution solvent is varied over time. As the elution solvent passes through column 220, it selectively removes compounds from the beads at different times. A first alliquot is collected in vial A, a second in vial B, a third in vial C, and so forth, as the pH of the elution solvent is varied. Thus, after the compounds of interest have been eluted from the column, each vial contains a different species that may be the drug candidate.

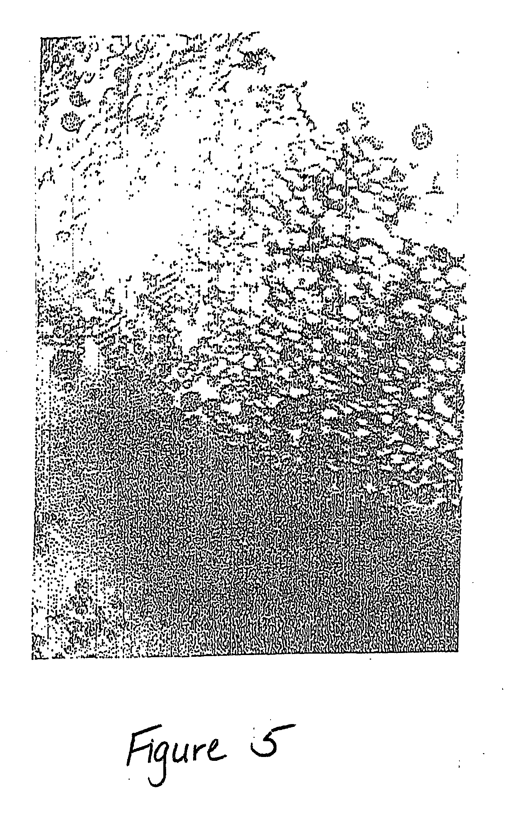

Figure 5 illustrates the same vials that are shown in Figure 4 after elution buffer has been exchanged for binding buffer and resin beads have been added to the vials. The beads are the same type of polystyrene resin beads as used in the separation process described above. Before the beads are added, the elution solvent is exchanged for a binding solvent over a dialysis membrane. After the exchange is complete, the beads may be added and the drug candidates are immobilized on the surface of the beads. A fluid containing suspended colloid particles including the immobilized target molecule of interest, is added to each of the vials. The beads are incubated for between 30 minutes and 2 hours. The vials may be agitated to promote binding of the binding partners. After an adequate incubation period, the beads contained in each of the vials are examined to ascertain whether or not they have been decorated by colloid particles. If any of the beads have been decorated, this may indicate that a binding event has occurred between the target molecule and a component of the original mixture. This interaction may indicate that the component can be used as a drug to block the biological activity of the target molecule. Alternatively, the component can be used in a diagnostic assay to differentiate between a healthy state and a disease state that has been correlated with the presence of the target protein.

### Comparative Example 7 (Prophetic)

The following is an example of how the methods described herein may be used in an integrated approach to diagnose and treat disease. It is known that cells associated with cancer often exclusively express or over express certain receptors on their surfaces. These cancer-associated cell surface receptors are known as tumor markers. Blocking the action of tumor markers, such as HER/2neu, with molecules that tightly bind to them has been shown to slow or eliminate tumor growth. Although a few tumor markers have been identified, researchers theorize that many more exist, but as yet remain unknown. Methods described herein may be used to detect tumor markers and also to screen for drugs to block their activity. One method of performing such an assay is as follows.

The cell surface receptors, known as tumor markers, interact with unknown ligands in the body. Cells or cell membranes may be lysed, fractionated and the contents attached to SAM-modified colloids as described herein. The colloids may or may not be derivatized to also present auxiliary signaling moieties. Healthy cells and disease-associated cells would be separately incubated with identical aliquots from batches of the candidate ligand-bearing colloids. One can then look for a population of colloids that bind differentially to disease-associated cells when compared to the binding to non-disease-associated cells. This differential binding implies that the colloid immobilized ligand is a binding partner of a tumor marker on the cell.

A reserved portion of the fraction that was attached to the colloids may then be analyzed to identify and characterize the binding partner of the tumor marker. Colloids derivatized with the binding partner might be used in diagnostic assays to signal the presence of disease or the binding partner may be used as a therapeutic to block the biological activity of the tumor marker.

Alternatively, the binding partner isolated from the lysate can be used in various drug screening strategies, including the following. Healthy and disease-related cells may be incubated with drug candidates and colloids bearing the binding partner. A loss of colloid binding to the cell may indicate that the candidate drug either bound directly to the tumor marker or indirectly affected its expression at the cell surface. In an in vitro drug screening assay, colloids bearing the binding partner may be incubated in solutions containing candidate drugs free in solution or attached to surfaces which can be, for example, flat substrates or particles such as beads or colloids. Detection methods described herein and in International Pat Apl. Ser. No: PCT/US00/01997, filed 01125/00, and in International Pat. Apl. Ser. No: PCT/US00/01504, filed 01/21/00, and in commonly-owned, copending U.S. Pat. Apl. Ser. No. 09/631,818, entitled "Rapid and Sensitive Detection of Protein Aggregation," by Bamdad et al., and in commonly-owned, copending U.S. Pat Apl. Ser. No. 09/602,778 entitled "Interaction of Colloid-Immobilized Species with Species on Non-Colloidal Structures," by Bamdad et al., may then be used to identify drug candidates that are bound to the colloid-bound binding partner. For example, fractions from a soil sample could be attached to colloids via EDC/NHS coupling and added separately to solutions containing colloids bearing the binding partner for the tumor marker. Solutions that contained elements that interacted with the tumor marker's binding partner would turn from pink to blue. Alternatively, drug candidates may be synthesized by combinatorial methods onto beads. These beads, each bearing a different drug candidate could then be incubated with the colloids bearing the tumor marker's binding partner. In this case, binding between the species on the colloid and the species on the bead could be detected via a signaling element on the colloid or by visually observing the aggregation of colloids onto the bead presenting the cognate drug.

### Comparative Example 8 (Prophetic)

Proteins can be characterized according to their ability to bind to certain known species such as chemical compounds, proteins, peptides, interaction domains and nucleic acids. Determining the binding capability of protein is an integral part of assigning function and determining the potential of the protein of interest as a therapeutic target. The number of potential protein binding partners of any particular protein of interest is large and therefore cannot be conveniently displayed on, for example, a chip surface. However, protein interactions are often mediated by recurrent recognition motifs, or domains, which occur in several different proteins. Protein interaction motifs typically retain their functional structure even when expressed as discrete units, rather than in the context of the parent protein and can therefore be synthesized or expressed as independent functional units and immobilized on chips. Important clues as to the function of an uncharacterized protein can be gleaned from determining the protein's interactions with other biomolecules and specifically with recognition motifs within those proteins.

A variety of protein interaction domains or recognition motifs have been reported in the literature and are known to those skilled in the art. Examples of protein interaction modules, which can be used to characterize proteins, for example, as part of a "protein function chip," are described below.

Discrete protein interaction motifs are exemplified by Src homology 2 domains (SH2), which recruit cytoplasmic signaling proteins. Grb-2 and Nck, for example, contain SH2 and SH3 domains, which recognize proline-rich sequences and act as adaptor proteins to allow additional proteins to be recruited to activated receptor tyrosine kinases. PDZ domains bind to certain C-terminal sequences on the inner face of receptors and are believed to be important in receptor clustering. PTB domains are similar to SH2 domains, but bind to different specific residues surrounding pY-containing motifs. FHA domains bind to phosphothreonine-containing peptides, while 14-3-3 domains bind to phosphoserine-containing motifs. PH domains bind to phosphoinositides, allowing membrane-association of signaling proteins. FYVE domains also bind to lipid headgroups, such as PI₃P. The Death domain, DED, BAD and CARD domains are motifs that are integral components of protein-protein interactions that trigger apoptosis. Functional domains or binding motifs include, but are not limited to, Death domains, SH2 and SH3, kringle domains, RGD motifs, acidic activation domains, and polyglutamine repeats, armadillo motifs, Grb 2 domains, enzyme substrates, cytoplasmic tails of proteins, MUC1 repeats, WW motifs and polyproline sequences. Of course, there are other domains or motifs. '

Devices may be configured to characterize a plurality of molecular interactions in a single procedure. For instance, surfaces such as protein array chips with one or more spatially addressable regions can be generated wherein each spatially addressable region displays a different immobilized species, such as a protein recognition module. An immobilized species, such as an uncharacterized protein, can be attached to colloid particles and then incubated with the chip. The spatial addresses to which the protein of interest binds can be discerned by a variety of techniques. For example, the agglomeration of colloids in a particular region can render that location red. Alternatively, the colloids may be derivatized with a signaling entity, such as an electro-active moiety for use with functional domain chips formed on electrodes. In addition, colloids can be constructed of or modified to include optically emissive compounds such as fluorescent moieties or fluorescent proteins. Following an incubation period, the chip surface is rinsed to remove unbound colloid particles, and the spatial pattern of the residual fluorescence can reveal the identity of the motifs to which the protein of interest has bound. Bound colloidal particles can be detected visually or with the aid of instrumentation, for example, electrodes or optical detectors. These and other techniques may provide for automation of part, or all, of the characterization process.

Minimal interaction motifs typically retain their functional structure and can therefore be synthesized or expressed as independent units and immobilized on surfaces such as chips or particles. For convenience, these motifs can be modified with affinity tags to facilitate binding to surfaces that display binding partners for these tags. For example, NTA-Ni(II) self-assembled monolayers can be generated on gold-coated substrates to immobilize motifs that have been modified with histidine tags. Masking, micro contact printing or physical isolation techniques, i.e. hydrophilic vs. hydrophobic, can be used to attach separate affinity tagged species to different regions on a surface.

Surfaces can be incubated with components other than proteins, including but are not limited to, peptides, fragments or mixtures of proteins, nucleic acids and complexes thereof, chemicals, drug candidates, drugs and cells. These components may be in purified form or derived from cell-lysates and fractions thereof, genomic DNA, cDNA libraries, drug or compound libraries, natural products samples, and the like.

### Comparative Example 9 (Prophetic)

A variation of this technique can be used to identify critical functionalities that are lost in a specific disease state. In this case, a chip presenting a library of functional modules is incubated with a mixed pool of proteins that have been modified to facilitate the attachment of a colloid particle that also bears a signaling capability such as an optically emissive moiety. Components of the protein pool are allowed to interact with surface-immobilized modules such that binding occurs. Colloids are then added and allowed to bind to the proteins that have interacted with the immobilized species. For example, the protein pool may have been expressed with a histidine tag such that NTA-Ni-presenting colloids can bind to them. After a wash step, a 3-dimensional interaction map is generated by measuring optical emission at each location of the spatially addressable surface. To determine critical functionalities that are lost in a specific disease state, the 3-D interaction map from a healthy set of cells or tissues is compared to the interaction map generated by the binding of proteins derived from cells or tissues present in a particular disease state.

### Example 2 (Prophetic)

In the invention, components, in solution, can be allowed to interact with a spatially addressable surface that presents a set of putative binding partners. A level of interaction is detected at each spatial location to generate an interaction pattern, or map. For example, a spatially addressable chip is derivatized to present a biomolecular array. A set of components in solution is allowed to interact with the surface and the level of bound species at each location is determined to generate an interaction pattern. Species immobilized in the array are proteins or protein interaction motifs; species present in solution may be comprised of proteins, protein fragments, protein interaction domains or motifs, nucleic acids, drugs or drug candidates.

As a comparative example, chemical compounds can be characterized in this way. For example, compounds, which may be drug candidates, are allowed to interact with a protein motif chip. The resultant interaction pattern is a fingerprint of the activity of the compound. Libraries of interaction patterns can be generated to fingerprint the activity of known drugs. Interaction patterns are then generated for as yet uncharacterized compounds. These novel interaction patterns are then compared to the reference interaction patterns, or fingerprints, of known drugs to get an indication of the activity of the uncharacterized compound.

It is possible to determine how the activity profile of a cell is altered in response to treatment with a drug candidate. Cellular components may be exposed to a drug and then allowed to interact with the spatially addressable chip that presents putative binding partners. In this case the resultant interaction pattern, when compared to the interaction pattern in the absence of drug, reflects changes in the binding pattern of the cellular components. In the invention, these cellular component interaction patterns that reflect differential binding effects due to drug treatment can be used to compare the activity of a known drug to an unknown drug candidate.

As a comparative example, compound drug libraries can be characterized by determining the effect of a drug on the ability of two or more other components to interact, for example, the binding of cellular products to interaction motifs. For instance, proteins derived from a tumor cell line are attached to signaling colloids and incubated with a surface, such as an interaction chip, displaying immobilized motifs that have been linked to cancer. The 3-dimensional interaction landscape is then compared to the interaction pattern generated after the cellular products have been treated with drug candidates. This can facilitate the rapid characterization of compound libraries for their ability to inhibit, enhance or otherwise alter certain interactions or classes of interactions. Characterization may be performed de novo or, in accordance with the invention, by comparing the interaction pattern generated by cellular products treated with an uncharacterized drug to interaction patterns generated by known drugs or other biologically active compounds. In this way, one can determine that a drug candidate is, for example, "taxol-like" or "endostatin-like", based on comparing its binding interactions to those of known drugs. Interaction chips can present proteins, nucleic acids, peptides, drugs, small molecules and the like. In the invention, they present proteins or protein interaction motifs, but it is not intended that the present invention be limited to the immobilization of particular interaction motifs or proteins implicated in any one disease.

As a comparative example, interaction patterns can be generated between surface-immobilized species and cellular components, which may be proteins, to delineate differences between the binding pattern of components produced by a healthy cell relative to that of a disease cell or to determine similar protein targets among a number of diseases. Cellular proteins may be incubated with the chips separately or together. That is to say, proteins may be separately attached to signaling colloids and sequentially incubated with the chips to generate interaction patterns that are unique to specific gene products. Alternatively, a cDNA library, or subset thereof, encoding a set of gene products is inserted into an expression vector designed to facilitate attachment of the expressed proteins to signaling colloids. Proteins can be grown up and expressed in host cells. In some cases, it may be desireable for the host cell's native proteins to interact with the proteins encoded by the cDNA library. For these cases, the cDNA's would be inserted into a regulatable expression vector such as the commercially available tetracycline inducible expression vector. In this way, conditions can be determined such that the levels of the cDNA proteins are comparable to the levels of the host cell's proteins.

Prior to colloid attachment, the expressed proteins are incubated with interaction chips. Groups of proteins will accumulate on spatial addresses that display a motif or compound that they recognize. Surfaces are rinsed and signaling colloids are then added. The number of colloids that attach to proteins at each site is proportional to the number of proteins that have bound to the entity displayed at that location.

The invention anticipates the detection of the interaction pattern using techniques that do not incorporate colloids. These include but are not limited to surface plasmon resonance (SPR), quartz crystal microbalance (QCM), as well as methods that label components that may interact with the array chip. For example, a cDNA library can be generated such that each protein component is expressed as a green fluorescent protein (GFP) fusion protein. In this way the fluorescence associated with the bound protein is directly detectable.

### Comparative Example 10

According to the following method intense signal amplification can be achieved. For example, an immobilized component can be provided on a surface, such as a protein chip, by attaching a first interacting species to the surface. An immobilized species, such as a putative binding partner, is attached to a colloid particle that also includes a linking entity. Colloid particles are then incubated on the surface so that the immobilized species may interact with the immobilized component on the surface. Prior to rinsing, a cross-linking compound that includes two or more binding sites for interacting with the linking entity can be added. The cross-linking compound may also include a signaling entity. The cross-linking compound can then form a network by joining a number of colloid particles together by binding with the linking entity on two or more colloid particles. After allowing adequate time for the cross-linking to occur, any unbound colloid particles, unattached colloid networks, and unbound cross-linking compounds can be rinsed from the surface. Any colloid particles bound to the surface may then be detected by directly observing networks of colloid particles or by detecting the presence, absence or relative intensity of either the colloid particles or the cross-linking compound.

Alternatively, prior to adding the cross-linking compound, unbound colloids may be rinsed from the surface. A second type of colloid particle including a linking entity, and optionally a signaling entity (but not the immobilized species), may then be added. A cross-linking compound capable of linking together colloid particles of either type is also added. The cross-linking compound may include a signaling entity. Networks of colloids are then developed on the surface, some of which may be bound to the surface via an interaction between the immobilized component and the immobilized species. Unbound materials may then be rinsed off and the binding event may be detected as described above.

A variety of linking entity/cross-linking compound pairs may be used. The cross-linking compound should be multivalent and should be able to bind two or more linking entities that have been attached to different colloid particles. Preferably, the cross-linking compound is attached to a flexible linker and may be incorporated into a polymer to aid in, for example, avoiding any steric hindrance effects that may occur between two adjacent colloid particles. The linking entity need only have one site for binding with a cross-linking compound and preferably is easily attached to a colloidal particle. Some examples of linking entity/cross-linking compound pairs are biotin/streptavidin, histidine tagged peptides/NTA/Ni(II) and DNA/DNA.

Accordingly, a first immobilized component may be attached to a protein chip surface. A putative binding partner is attached to a gold colloid particle as an immobilized species and the colloid particle also bears a biotin moiety. The colloid particles are then incubated with the protein chip. Without rinsing the chip surface, fluorescently labeled streptavidin, which has four binding sites for biotin, is added. The streptavidin provides a detectable fluorescent signal and also serves to cross-link a network of colloids, resulting in a network of colloids bound to the surface by as few as a single protein/protein interaction. Incubation time may be varied to allow the network to grow to a detectable size. After the network has been formed, any unbound colloids, as well as unbound colloid networks, can be rinsed from the surface. Any remaining networks of colloid particles and fluorescently labeled streptavidin serve to greatly amplify the signal, which may be detected by a fluorescence detector.

The fluorescent moiety can be genetically engineered, but need not be. For instance, any fluorophore can be used and may be attached using a number of methods, including via a thiol to form a SAM. In this way, the cumbersome and sometimes interfering step of genetically engineering a signaling entity to a protein of interest is avoided. Additionally, the ability to attach signaling entities to a generic support particle, allows for the "attachment" of a signaling element to any molecule, proteinaceous or synthetic. Furthermore, the fluorophore need not be directly molecularly bonded to the molecule of interest and therefore the activity of the molecule of interest may be less likely to be altered by the presence of the fluorescent label.

Those skilled in the art would readily appreciate that all parameters listed herein are meant to be exemplary and that actual parameters will depend upon specific application for which the methods of the present invention are used. It is, therefore, to be understood that the foregoing embodiments are presented by way of example only and that, within the scope of the appended claims, the invention may be practiced otherwise then as specifically described.

## Claims

1. A method of comparing the activities of a first drug or drug candidate and a second drug or drug candidate, comprising:
providing a first surface having a plurality of individually spatially addressable regions, wherein a different protein or protein interaction motif is fastened to each of the regions;
treating a first sample comprising cellular components with the first drug or drug candidate;
exposing the treated first sample to the first surface, and allowing interaction to occur between the components and each of the regions, such that at least one of the components binds to at least one of the protein or protein interaction motifs, thereby defining a first interaction pattern between the components and each of the regions;
determining the first interaction pattern;
providing a second surface having a plurality of individually spatially addressable regions that are identical to those of the first surface;
treating a second sample comprising cellular components, which are identical to those of the first sample, with the second drug or drug candidate;
exposing the treated second sample to the second surface, and allowing interaction to occur between the components and each of the regions, such that at least one of the components binds to at least one of the protein or protein interaction motifs, thereby defining a second interaction pattern between the components and each of the regions;
determining the second interaction pattern; and
comparing the first and second interaction patterns.

2. A method as claimed in claim 1, wherein a different protein interaction motif is fastened to each of the regions of the first and second surface.

3. A method as claimed in claim 1 or 2, wherein the cellular components are proteins.

4. A method as claimed in claim 3, wherein the cellular components are **uncharacterised** proteins.

5. A method as claimed in claim 3 or 4, wherein the cellular components are proteins expressed from a cDNA library.

6. A method as claimed in any of claims 3 to 5, wherein the cellular components are proteins derivatized with affinity tags.

7. A method as claimed in claim 6, wherein the cellular components are proteins derivatized with histidine tags, GST or streptavidin.

8. A method as claimed in any of the preceding claims, wherein the first and second surfaces are microtitre plates, chips, glass plates or electrodes.

9. A method as claimed in claim 8, wherein the first and second surfaces are gold-coated.

10. A method as claimed in any of the preceding claims, wherein the first and second surfaces comprise a SAM.

11. A method as claimed in any of the preceding claims, wherein the interaction patterns are determined by QCM.

12. A method as claimed in any of claims 1 to 10, wherein the interaction patterns are determined by SPR.

13. A method as claimed in any of claims 1 to 10, wherein the interaction patterns are determined by detecting signals at the surface regions, wherein the signals are light emission.

14. A method as claimed in claim 13, wherein the cellular components are proteins expressed from a cDNA library as GFP fusion proteins.

15. A method as claimed in any of claims 1 to 10, wherein the interaction patterns are determined by detecting signals at the surface regions, wherein the signals are electrical.

16. A method as claimed in any of the preceding claims, wherein the cellular components are proteins derivatized with affinity tags to facilitate the binding of colloids, and the determining steps involve immobilizing colloids to the bound cellular components.

17. A method as claimed in claim 16, wherein the cellular components are proteins expressed from a cDNA library with histidine tags, and the colloids are NTA-Ni-presenting colloids.

18. A method as claimed in claim 16 or 17, wherein the colloids carry auxiliary signalling entities.

19. A method as claimed in claim 18, wherein the auxiliary signalling entities are fluorescent.

## Patentansprüche

1. Verfahren zum Vergleich der Aktivitäten eines ersten Arzneimittels oder Arzneimittelkandidaten und eines zweiten Arzneimittels oder Arzneimittelkandidaten, umfassend:
Bereitstellen einer ersten Oberfläche mit einer Vielzahl individuell räumlich adressierbarer Regionen, worin ein unterschiedliches Protein- oder Proteininteraktionsmotiv an jeder der Regionen befestigt ist;
Behandeln einer ersten Probe, umfassend Zellkomponenten,
mit dem ersten Arzneimittel oder Arzneimittelkandidaten;
Exponieren der behandelten ersten Probe gegenüber der ersten Oberfläche, und Zulassen des Auftretens einer Interaktion zwischen den Komponenten und jeder der Regionen dergestalt, dass mindestens eine der Komponenten an mindestens eines der Protein- oder Proteininteraktionsmotive bindet, wobei ein erstes Interaktionsmuster zwischen den Komponenten und jeder der Regionen definiert ist;
Bestimmen des ersten Interaktionsmusters;
Bereitstellen einer zweiten Oberfläche mit einer Vielzahl individuell räumlich adressierbarer Regionen, die mit denen der ersten Oberfläche identisch sind;
Behandeln einer zweiten Probe, umfassend Zellkomponenten, die mit denen der ersten Probe identisch sind, mit dem zweiten Arzneimittel oder Arzneimittelkandidaten;
Exponieren der behandelten zweiten Probe gegenüber der zweiten Oberfläche, und Zulassen des Auftretens einer Interaktion zwischen den Komponenten und jeder der Regionen dergestalt, dass mindestens eine der Komponenten an mindestens eines der Protein- oder Proteininteraktionsmotive bindet, wobei ein zweites Interaktionsmuster zwischen den Komponenten und jeder der Regionen definiert ist;
Bestimmen des zweiten Interaktionsmusters; und
Vergleichen der ersten und zweiten Interaktionsmuster.

2. Verfahren nach Anspruch 1, worin ein unterschiedliches Proteininteraktionsmotiv an jeder der Regionen der ersten und der zweiten Oberfläche befestigt ist.

3. Verfahren nach Anspruch 1 oder 2, worin die Zellkomponenten Proteine darstellen.

4. Verfahren nach Anspruch 3, worin die Zellkomponenten nicht charakterisierte Proteine darstellen.

5. Verfahren nach Anspruch 3 oder 4, worin die Zellkomponenten aus einer cDNA-Bibliothek exprimierte Proteine darstellen.

6. Verfahren nach einem der Ansprüche 3 bis 5, worin die Zellkomponenten mit Affinitätstags derivatisierte Proteine darstellen.

7. Verfahren nach Anspruch 6, worin die Zellkomponenten mit Histidintags, GST oder Streptavidin derivatisierte Proteine darstellen.

8. Verfahren nach einem der vorangehenden Ansprüche, worin die ersten und zweiten Oberflächen Mikrotiterplatten, Chips, Glasplatten oder Elektroden darstellen.

9. Verfahren nach Anspruch 8, worin die ersten und zweiten Oberflächen goldbeschichtet sind.

10. Verfahren nach einem der vorangehenden Ansprüche, worin die ersten und zweiten Oberflächen eine SAM umfassen.

11. Verfahren nach einem der vorangehenden Ansprüche, worin die Interaktionsmuster mittels QCM bestimmt werden.

12. Verfahren nach einem der Ansprüche 1 bis 10, worin die Interaktionsmuster mittels SPR bestimmt werden.

13. Verfahren nach einem der Ansprüche 1 bis 10, worin die Interaktionsmuster durch Nachweissignale an den Oberflächenregionen bestimmt werden, worin die Signale eine Lichtemission darstellen.

14. Verfahren nach Anspruch 13, worin die Zellkomponenten Proteine darstellen, die aus einer cDNA-Bibliothek als GFP-Fusionsproteine exprimiert werden.

15. Verfahren nach einem der Ansprüche 1 bis 10, worin die Interaktionsmuster durch Nachweissignale an den Oberflächenregionen bestimmt werden, worin die Signale elektrisch sind.

16. Verfahren nach einem der vorangehenden Ansprüche, worin die Zellkomponenten mit Affinitätstags derivatisierte Proteine zur Förderung der Bindung von Kolloiden darstellen und die Bestimmungsschritte das Immobilisieren von Kolloiden an den gebundenen Zellkomponenten beinhalten.

17. Verfahren nach Anspruch 16, worin die Zellkomponenten Proteine darstellen, die aus einer cDNA-Bibliothek mit Histidintags exprimiert werden, und die Kolloide NTA-Nipräsentierende Kolloide darstellen.

18. Verfahren nach Anspruch 16 oder 17, worin die Kolloide Hilfssignalisierungsentitäten tragen.

19. Verfahren nach Anspruch 18, worin die Hilfssignalisierungsentitäten fluoreszieren.

## Revendications

1. Méthode de comparaison des activités d'un premier médicament ou médicament candidat et d'un second médicament ou médicament candidat, qui comprend les étapes consistent :
à produire une première surface comportant une pluralité de régions pouvant être individuellement et spatialement ciblées, où une protéine différent ou un motif d'interaction protéique est rattaché à chacune des régions ;
à traiter un premier échantillon comprenant des composants cellulaires avec le premier médicament ou médicament candidat ;
à exposer le premier échantillon traité à la première surface et à laisser l'interaction avoir lieu entre les composants et chacune des régions de façon à ce qu'au moins un des composants se lie à au moins la protéine ou à des motifs d'interaction protéique, définissant ainsi un premier modèle d'interaction entre les composants et chacune des régions ;
à déterminer le premier modèle d'interaction ;
à produire une seconde surface comportant une pluralité de régions pouvant être individuellement et spatialement ciblées qui sont identiques à celles de la première surface ;
à traiter un second échantillon comprenant des composants cellulaires qui sont identiques à ceux du premier échantillon avec le second médicament ou médicament candidat ;
à exposer le second échantillon traité à la seconde surface et à laisser l'interaction avoir lieu entre les composants et chacune des régions de façon à ce qu'au moins un des composants se lie à au moins la protéine ou à des motifs d'interaction protéique, définissant ainsi un second modèle d'interaction entre les composants et chacune des régions ;
à déterminer le second modèle d'interaction ; et
à comparer les premier et second modèles d'interaction.

2. Méthode selon la revendication 1, où un motif d'interaction protéique différent est rattaché à chacune des régions des première et seconde surfaces.

3. Méthode selon la revendication 1 ou 2, où les composants cellulaires sont des protéines.

4. Méthode selon la revendication 3, où les composants cellulaires sont des protéines non **caractérisées**.

5. Méthode selon la revendication 3 ou 4, où les composants cellulaires sont des protéines exprimées à partir d'une banque d'ADNc.

6. Méthode selon l'une quelconque des revendications 3 à 5, où les composants cellulaires sont des protéines fonctionnalisées avec des marqueurs d'affinité.

7. Méthode selon la revendication 6, où les composants cellulaires sont des protéines fonctionnalisées avec des marqueurs histidine, GST ou streptadivine.

8. Méthode selon l'une quelconque des revendications précédentes, où les première et seconde surfaces sont des plaques de microtitrage, des puces, des plaques de verre or des électrodes.

9. Méthode selon la revendication 8, où les première et seconde surfaces sont recouvertes d'or.

10. Méthode selon l'une quelconque des revendications précédentes, où les première et seconde surfaces comprennent une monocouche auto-assemblée (SAM).

11. Méthode selon l'une quelconque des revendications précédentes, où les modèles d'interaction sont déterminés par microbalance à cristal de quartz (QCM).

12. Méthode selon l'une quelconque des revendications 1 à 10, où les modèles d'interaction sont déterminés par résonance des plasmons de surface (SPR).

13. Méthode selon l'une quelconque des revendications 1 à 10, où les modèles d'interaction sont déterminés par détection des signaux au niveau des régions de la surface, lesdits signaux correspondant à une émission de lumière.

14. Méthode selon la revendication 13, où les composants cellulaires sont des protéines exprimées à partir d'une banque d'ADNc sous la forme de protéines de fusion avec la GFP.

15. Méthode selon l'une quelconque des revendications 1 à 10, où les modèles d'interaction sont déterminés par détection des signaux au niveau des régions de la surface, lesdits signaux étant électriques.

16. Méthode selon l'une quelconque des revendications précédentes, où les composants cellulaires sont des protéines fonctionnalisées avec des marqueurs d'affinité pour faciliter la liaison de colloïdes, et où les étapes de détermination font intervenir l'immobilisation de colloïdes sur les composants cellulaires liés.

17. Méthode selon la revendication 16, où les composants cellulaires sont des protéines exprimées à partir d'une banque d'ADNc avec des marqueurs histidine, et où les colloïdes sont des colloïdes présentateurs d'un groupement NTA-Ni.

18. Méthode selon la revendication 16 ou 17, où les colloïdes portent des entités de signalisation auxiliaires.

19. Méthode selon la revendication 18, où les entités de signalisation auxiliaires sont fluorescentes.
